(19) Europäisches Patentamt
European Patent Office
Office européen des brevets

(11) **EP 2 130 581 B1**

(12) **EUROPEAN PATENT SPECIFICATION**

(45) Date of publication and mention of the grant of the patent:
**09.01.2013 Bulletin 2013/02**

(21) Application number: **08739895.4**

(22) Date of filing: **04.04.2008**

(51) Int Cl.:
*B01J 20/26* (2006.01)   *A61F 13/15* (2006.01)
*A61F 13/49* (2006.01)   *A61F 13/53* (2006.01)
*C08F 2/10* (2006.01)   *C08F 2/48* (2006.01)
*C08F 20/06* (2006.01)   *C08J 3/12* (2006.01)

(86) International application number:
**PCT/JP2008/056789**

(87) International publication number:
**WO 2008/126793 (23.10.2008 Gazette 2008/43)**

(54) **GRANULAR WATER ABSORBENT COMPRISING WATER ABSORBING RESIN AS THE MAIN COMPONENT**

GRANULARES WASSERABSORPTIONSELEMENT MIT WASSERABSORBIERENDEM HARZ ALS HAUPTBESTANDTEIL

ABSORBANT D'EAU GRANULAIRE COMPRENANT UNE RÉSINE D'ABSORPTION D'EAU EN TANT QUE COMPOSANT PRINCIPAL

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **05.04.2007   JP 2007099041**

(43) Date of publication of application:
**09.12.2009 Bulletin 2009/50**

(73) Proprietor: **Nippon Shokubai Co., Ltd.**
**Osaka-shi, Osaka 541-0043 (JP)**

(72) Inventors:
• **ADACHI, Yoshifumi**
**Himeji-shi**
**Hyogo 672-8023 (JP)**
• **TAHARA, Ryoko**
**Kitaibaraki-shi**
**Ibaraki 319-1543 (JP)**

• **WADA, Katsuyuki**
**Himeji-shi**
**Hyogo 670-0081 (JP)**

(74) Representative: **Mai, Dörr, Besier**
**Patentanwälte**
**Steuerberater/Wirtschaftsprüfer**
**John-F.-Kennedy-Straße 4**
**65189 Wiesbaden (DE)**

(56) References cited:
EP-A1- 0 467 073     WO-A1-2005/075070
JP-A- 2000 000 463   JP-A- 2001 234 087
JP-A- 2003 195 883   JP-A- 2005 111 474
JP-A- 2005 288 265   JP-A- 2005 344 103
JP-A- 2006 028 481   JP-A- 2006 057 075
JP-A- 2006 116 535   US-A1- 2005 221 980
US-B2- 6 906 159

EP 2 130 581 B1

**Description**

TECHNICAL FIELD

The present invention relates to a particulate water-absorbing agent having a water-absorbing resin as a main component, which agent is excellent in absorbency irrespective of a practical urine type and has decreased re-wetness, an absorbing goods containing the particulate water-absorbing agent, and a method for production of the particulate water-absorbing agent.

BACKGROUND ART

At present, as a material constituting an absorbing goods such as a pet sheet, a disposable diaper, an incontinent pad, a water-absorbing agent having a water-absorbing resin as a main component has been widely used, as well as hydrophilic fiber such as pulp.

Such a water-absorbing resin is a water-swelling and water-insoluble cross-linked polymer, and it has been said to be important to absorb a large quantity of physiological saline. With functional enhancement of the absorbing goods, many improvements have been proposed. In recent years, in the absorbing goods, improvement to make possible use for a long period of time (for example, 16 hours at night), or improvement to produce the absorbing goods in a lower cost has been carried out. In such a trend, needs to improve absorption performance have been increasing more than ever, such as higher absorbency has been required to the water-absorbing agent, or the like.

With functional enhancement of the absorbing goods (a disposable diaper), there have been filed many patent applications relating to parameter inventions specifying various properties of the water-absorbing agent (an absorbing resin), or production methods thereof.

For example, US-A-2005-0,221,980 has disclosed a water-absorbing agent excellent in absorption against pressure in any of NaCl solutions with different concentration. US-B-6,187,872 has disclosed a water-absorbing resin having an absorption against pressure of equal to or higher than 20 g/g and a small water-extractable content, obtained by neutralization of a polymer after polymerization of non-neutralized acrylic acid. JP-A-2006-122737 has disclosed a water-absorbing agent exerting high absorption performance even under any level of load. US-B-6,586,549 has disclosed a water-absorbing composition which exhibits a high absorption capacity, and exhibits excessive-liquid permeation buffering effects greatly in high concentration portions where particles of the water-absorbing composition closely cohere by adding an excessive-liquid permeation buffer to a water-absorbent resin. US-A-6,906,159 has disclosed a process for producing a water-absorbent resin which comprises polymerizing an aqueous solution of a monomer including acrylic acid and/or its sodium salt as major components, wherein (1) the aqueous solution has a monomer component concentration of not less than 45 weight %; (2) the polymerization is carried out while water is evaporated so that the ratio (concentration ratio) between a solid component concentration in a hydropolymer as formed by the polymerization and a solid component concentration in the aqueous monomer solution will not be less than 1.10; and (3) the solid component concentration in the hydropolymer as formed by the polymerization is not more than 80 weight %.

US 6,906,159 B2 discloses water absorbent resins having a high absorption capacity without load and a low extractable content. US 6, 906, 159 B2 does not disclose a water absorbing agent possessing an absorption capacity without load in artificial urine (A) of not less than 62 g/g.

DISCLOSURE OF INVENTION

Although as shown in the above various Patent Literatures, a number of methods for producing water-absorbing resins, water-absorbing resins, and parameters (fod controlling property), most of the above Patent Literatures aim at only enhancement of absorption performance to one kind of artificial urine such as physiological saline. In US-A-2005-0,221,980, an attempt has been made to enhance absorption against pressure by using solutions with different sodium chloride concentrations. In addition, USP-A-2005-0,221,980 has stated that ion strength is largely different among adult urine, infant urine and newborn urine, and pointed out that evaluation with only a conventional physiological saline (an aqueous 0.9% by weight sodium chloride solution) is insufficient. However, on the other hand, in the US-A-2005-0, 221, 980, even under recognition of such a state, evaluation was performed only with solutions having concentrations of sodium chloride varied simply. Practically, components in urine vary in a large degree, depending on animal kind, or growth degree of an individual and environment, and many kinds of components such as urea or a magnesium component, a potassium component, an ammonium component, a phosphorous component, other than sodium chloride are contained. Therefore, satisfactory performance could not been attained in practical use of a disposable diaper or a pet sheet etc. for absorbing urine. In addition, by a method which comprises polymerizing non-neutralized acrylic acid and then neutralizing the polymer, as stated in US-B-6,187,872, a production process is complicated and a water-absorbing agent cannot been provided at a low price. Accordingly, the present invention has been proposed in view of the above circumstances. It is an object of the present invention to provide a water-absorbing goods having excellent absorption performance in a practical use, in low price. In addition, it is another object of the present invention to provide a water-absorbing agent required to provide such absorbing goods.

The present inventors have intensively studied a way to solve the above problems, to find that by using a water-absorbing agent having specific parameters to a plurality of artificial urines having different ion strengths or dissolving components, an absorbing goods having excellent performance irrespective of urine kinds, when absorbed with a practical urine as in practical use, can be provided, to specify a structure which a water-absorbing agent should have for obtaining such a water-absorbing agent. Based on the knowledge, the present invention has been accomplished.

Specifically, a particulate water-absorbing agent provided by the present invention is particulate water-absorbing agent comprising a polyacrylate salt-type water-absorbing resin as a main component, and satisfying the following (a) and (b):

(a) absorption capacity without load in artificial urine (A) is equal to or higher than 62 g/g; and

(b) an increase rate of absorption capacity without load in artificial urine (B) relative to artificial urine (A) is equal to or larger than 20%, wherein the polyacrylate salt-type water-absorbing resin is a cross-linked polymer containing acrylic acid (salt) in the repeating units thereof (excluding a cross-linking agent) in an amount of 70 to 100% by mole, and the acrylic acid (salt) as the repeating unit is neutralized in the range of 20 to 100% by mole in the form of an alkali metal salt.

Preferably, the particulate water-absorbing agent provided by the present invention is a particulate water-absorbing agent further satisfying the following (d) :

(d) weight average molecular weight (Mw) of the main chain of the water-absorbing resin is equal to or higher than 1,000,000 and molecular weight distribution (Mw/Mn) thereof is equal to or smaller than 3.3.

In a particulate water-absorbing agent relevant to the present invention, it is preferable that ratio of particles having a particle diameter of equal to or larger than 300 $\mu$m and smaller than 600 $\mu$m is equal to or larger than 50% by weight.

It is preferable that the particulate water-absorbing agent according to the present invention has an extractable content of 20 to 70% by weight.

It is preferable that the particulate water-absorbing agent according to the present invention has logarithmic standard deviation ($\sigma\zeta$) of particle size distribution of 0.20 to 0.45.

It is preferable that the particulate water-absorbing agent according to the present invention satisfies the following (e) to (g):

(e) weight average particle diameter (D50) is 200 to 500 $\mu$m;

(f) an amount of particles smaller than 150 $\mu$m as determined by a JIS standard sieve is 0 to 5% by weight; and

(g) an amount of particles equal to or larger than 850 $\mu$m as determined by a JIS standard sieve is 0 to 5% by weight.

It is preferable that the particulate water-absorbing agent according to the present invention further comprises a chelating agent in an amount of 0.00001 to 10 parts by weight, relative to 100 parts by weight of the water-absorbing resin.

It is preferable that the polyacrylate salt-type water-absorbing resin contained in the particulate water-absorbing agent according to the present invention is a water-absorbing resin obtained by polymerization of a monomer under conditions satisfying the following (h) and (i):

(h) the monomer contains acrylic acid (a salt thereof) in an amount of 70 to 100% by mole as a monomer, and a cross-linking agent in an amount of 0.000 to 1.000% by mole relative to the monomer; and

(i) a concentration of an aqueous solution of the monomer is equal to or higher than 40% by weight, wherein the polyacrylate salt-type water-absorbing resin is a cross-linked polymer containing acrylic acid (salt) in the repeating units thereof (excluding a cross-linking agent) in an amount of 70 to 100% by mole, and the acrylic acid (salt) as the repeating unit is neutralized in the range of 20 to 100% by mole in the form of an alkali metal salt.

It is preferable that the polyacrylate salt-type water-absorbing resin contained in a particulate water-absorbing agent according to the present invention is a water-absorbing resin obtained by cast polymerization of the aqueous solution of the monomer while controlling a polymerization initiation temperature at equal to or higher than 40°C, or a peak temperature in polymerization at equal to or higher than 100°C, and

wherein the polyacrylate salt-type water-absorbing resin is a cross-linked polymer containing acrylic acid (salt) in the repeating units thereof (excluding a cross-linking agent) in an amount of 70 to 100% by mole, and the acrylic acid (salt) as the repeating unit is neutralized in the range of 20 to 100% by mole in the form of an alkali metal salt.

It is preferable that the particulate water-absorbing agent according to the present invention is obtained by agglomeration with water or an aqueous solution, wherein the amount of water or an aqueous solution to be used is in the range of 0.5 to 20 parts by weight, relative to 100 parts by weight of solid content of the water-absorbing resin, and amount of the hydrophilic organic solvent is in the range of 0 to 10 parts by weight, relative to the water-absorbing resin mixture.

The particulate water-absorbing agent of the present invention can be produced at low cost, provide a absorbing goods, which can absorb variety of excretes from animals including human, when used in the absorbing goods, and can improve absorbing amount or re-wet in the absorbing goods.

BEST MODE FOR CARRYING OUT THE INVENTION

Explanation will be given below in detail on the present invention. A technical scope of the present invention, however, should be defined based on description of claims, and should not be limited by the following specific embodiments.

Explanation will be given below in detail on raw materials or reaction conditions etc. to be used in the particulate water-absorbing agent (hereafter also referred to simply as "a water-absorbing agent") relevant to the present invention.

(1) Water-absorbing agent

In the present invention, "a water-absorbing agent" is referred to as an absorbing gelling agent (also called as a solidifying agent) for an aqueous liquid having a water-absorbing resin as a main component. The aqueous liquid, as used herein, includes both water alone or a water mixture, so long as it contains water in a solid, liquid or vapor form. Typically, it may be used in water absorption of urine, in particular, human urine.

As described above, according to the present invention, a particulate water-absorbing agent is provided, which comprises a polyacrylate salt-type water-absorbing resin as a main component, and satisfies the following (a) and (b):

(a) absorption capacity without load in artificial urine (A) is equal to or higher than 62 g/g; and
(b) an increase rate of absorption capacity without load in artificial urine (B) relative to artificial urine (A) is equal to or larger than 20%, wherein the polyacrylate salt-type water-absorbing resin is a cross-linked polymer containing acrylic acid (salt) in the repeating units thereof (excluding a cross-linking agent) in an amount of 70 to 100% by mole, and the acrylic acid (salt) as the repeating unit is neutralized in the range of 20 to 100% by mole in the form of an alkali metal salt.

Preferably the particulate water-absorbing agent provided, further satisfies the following (d):

(d) weight average molecular weight (Mw) of the main chain of the water-absorbing resin is equal to or higher than 1,000,000 and molecular weight distribution (Mw/Mn) thereof is equal to or smaller than 3.3.

It should be noted that the particulate water-absorbing agent is specified by the parameters, and explanation will be given later on a specific preferable method for attaining these parameters.

The water-absorbing agent of the present invention may contain a component other than the water-absorbing resin as an additive. In view of water-absorbing characteristics, the water-absorbing resin is contained in the water-absorbing agent, in an amount of equal to or more than 70% by weight, preferably equal to or more than 80% by weight, and particularly preferably equal to or more than 85% by weight. The additives preferably used include a chelating agent, inorganic fine particles or water, which will be explained later.

(2) Polyacrylate salt-type water-absorbing resin

The water-absorbing resin, which composes the water-absorbing agent of the present invention, is a polyacrylate salt-type water-absorbing resin. A water-swelling and water-insoluble, cross-linked polymer containing acrylic acid (salt) in the repeating units thereof (excluding a cross-linking agent) in an amount of 70 to 100% by mole, preferably 80 to 100% by mole, and more preferably 90 to 100% by mole, is used.

The acrylic acid (salt) as the repeating unit is neutralized in the range of 20 to 100% by mole, preferably 50 to 99% by mole, and more preferably 60 to 90% by mole, in the form of an alkali metal salt, and preferably a sodium salt.

(3) Other monomers and cross-linking agent

Other unsaturated monomer may be used in an amount of 0 to 70% by mole, relative to the total amount of monomers. Typically, such an unsaturated monomer may include hydrophilic monomers such as methacrylic acid, maleic acid (anhydride), fumaric acid, crtonic acid, itaconic acid, vinyl sulfonic acid, 2-(meth)acrylamide-2-methylpropane sulfonic acid, (meth)acryloxyalkane sulfonic acid, N-vinyl-2-pyrrolidone, N-vinyl acetamide, (meth)acrylamide, N-isopropyl (meth)acrylamide, N,N-dimethyl (meth)acrylamide, 2-hydroxyethyl (meth)acrylate, methoxypolyethylene glycol (meth) acrylate, polyethylene glycol (meth)acrylate, and the salts thereof.

In addition, as the cross-linking agent which may be used, for example, one or more members of compounds which have at least two polymerizable double bonds in the molecule, such as N,N' -methylene bisacrylamide, (poly) ethylene glycol di (meth) acrylate, (poly)propylene glycol di (meth) acrylate, (polyoxyethylene) trimethylol propane tri (meth)acrylate, trimethylolpropane di(meth)acrylate, polyethylene glycol di($\beta$-acryloyloxypropionate), trimethylolpropane tri($\beta$-acryloyloxypropionate), poly(meth)aryloxyalkane; and compounds capable of forming a covalent bond by the reaction with a carboxyl group, such as polyglycidyl ether (like ethylene glycol diglycidyl ether), polyols (like ethylene glycol, polyethylene glycol, glycerin, sorbitol ) may be cited.

In the case of using a cross-linking agent, it is preferable to use essentially a compound having at least two polymerizable double bonds in its molecule, in consideration of absorbing characteristics of the obtained water-absorbing resin. Although the adding amount of the cross-linking agent is not especially limited, it is preferably in the range of 0.000 to 1.000% by mole, more preferably 0.001 to 0.100% by mole, still more preferably 0.002 to 0.05% by mole, particularly preferably 0.003 to 0.02% by mole, and most preferably 0.001 to 0.01% by mole relative to 100% by weight of the monomer(s), in view of physical properties.

In order to obtain the particulate water-absorbing agent of the present invention, the cross-linking agent is preferably used. More preferably, the compound having at least two polymerizable double bonds in its molecule is used, and (poly)ethylene glycol di(meth)acrylate or (poly)propylene glycol di(meth)acrylate is particularly preferably used.

In the present invention, there may be included, if necessary, a deodorant, an antibacterial agent, a fragrance, an inorganic powder such as silicon dioxide and titanium oxide, polysaccharides such as starch, cellulose and derivatives thereof, a hydrophilic polymer such as polyvinyl alcohol, a thermoplastic resin such as polyethylene and polypropylene, a foaming agent, a pigment, a dye, a hydrophilic staple fiber, a plasticizer, a chain transfer agent such as hypophosphorous acid (salt), in an amount of equal to or less than 5% by weight, preferably equal to or less than 1% by weight, relative to the total amount of the monomer(s).

(4) Polymerization step of water-absorbing resin

(Polymerization)

The polymerization in the present invention is not especially limited. Polymerization which is aqueous solution polymerization and cast polymerization (that is, cast polymerization in an aqueous solution) at a polymerization initiation temperature of equal to or higher than 40°C, or at a peak temperature of equal to or higher than 100°C, is preferably used. In the case where cast polymerization is not adopted, for example, in the case of reversed phase suspension polymerization or stirring polymerization in an aqueous solution (polymerization in a kneader as described in US-A-2004-110897, US-B-670,141, US-B-4,625,001, US-B-5,250, 640), or in the case where polymerization initiation temperature is below 40°C and peak polymerization temperature is below 100°C, molecular weight distribution of the main chain of the water-absorbing resin would be unduly broadened, which may not show stable absorbency when different artificial urines are used.

The reversed phase suspension polymerization is a polymerization method which comprises suspending of an aqueous solution of a monomer in a hydrophobic organic solvent. Such a method is described in U.S. Patents such as US-B-4,093,776, US-B-4,367,323, US-B-4,446,261, US-B-4,683,274, US-B-5,244,735, for example. The aqueous solution polymerization is a method for polymerizing an aqueous solution of a monomer without using a dispersion solvent. Such a method is described in U.S. Patents such as US-B-4,625,001, US-B-4,873,299, US-B-4,286,082, US-B-4,973,632, US-B-4,985,518, US-B-5,124,416, US-B-5,250,640, US-B-5,264,495, US-B-5,145,906, US-B-5,380,808, or in European Patents such as EP-B-0 811 636, EP-B-0 955 086, EP-B-0 922 717, for example. The monomers, cross-linking agents, polymerization initiators, and other additives described in these patents can be also applied, as appropriate, to the cast polymerization in an aqueous solution, which is a preferable embodiment of polymerization in the present invention.

A monomer concentration in a monomer solution in carrying out the polymerization is not especially limited. It is usually equal to or higher than 20% by weight, preferably equal to or higher than 30% by weight, further preferably equal to or higher than 35% by weight, more preferably equal to or higher than 40% by weight, and particularly preferably equal to or higher than 45% by weight. On the other hand, the upper limit of the monomer concentration is usually equal to or lower than 80% by weight, preferably equal to or lower than 70% by weight, and more preferably equal to or higher than 60% by weight. The monomer concentration below 20% by weight may cause inhibition of increase in absorption rate, while the monomer concentration over 80% by weight may tend to decrease absorbency.

(Polymerization apparatus)

The cast polymerization may be performed batchwise. The continuous cast polymerization using an endless belt (for example, US-A-2005-215,734) may be adopted preferably. The belt is preferably a belt made of a resin or rubber which is difficult to escape heat of polymerization from its contact face. In addition, in order to enhance absorption rate, an open space is preferably present at the upper part of a polymerization container. In addition, feeding thickness of the aqueous solution (or gel) of the monomer in supplying the aqueous solution of the monomer containing the monomer, polymerization initiator, cross-linking agent and dispersed solid onto the belt, is usually preferably 1 to 100 mm, more preferably 3 to 50 mm, and most preferably 5 to 30 mm. The thickness of the aqueous solution of the monomer below 1 mm would make temperature adjustment of the aqueous solution of the monomer difficult, while the thickness over 100 mm would make removal of heat of polymerization difficult, both of which would cause decrease in physical properties of the water-absorbing resin. Further, speed of the endless belt is usually preferably 0.3 to 100 m/minute, more preferably 0.5 to 30 m/minute, and most preferably 1 to 20 m/minute, although it depends on length of the polymerization apparatus. The belt speed slower than 0.3 m/minute would decrease productivity, while the belt speed faster than 100 m/minute would increase scale of the polymerization apparatus, both of which are not preferable.

Specifically, the polymerization apparatus to be used in the polymerization step to obtain the water-absorbing resin which composes the water-absorbing agent of the present invention, is preferably a continuous polymerization apparatus having an inlet of feeding an aqueous solution of monomer containing a monomer, a polymerization initiator, a cross-linking agent and a dispersed solid, an endless belt for conveying the monomer and a hydrate polymer formed therefrom, and an exit of discharging the hydrate polymer. A production method for obtaining the water-absorbing resin which composes the water-absorbing agent of the present invention, is preferably a continuous production method for the water-absorbing resin by using the continuous polymerization apparatus, wherein the lateral face and the ceiling plane of the continuous polymerization apparatus are covered, and have an apparatus space ratio, specified by the following formula 1, in the range of 1.2 to 20.

[Formula 1]

$$\text{[Formula 1]}$$

$$\text{Apparatus space ratio} = \text{B/A}$$

In the above formula, A (cm$^2$) represents a maximum cross-sectional area of the hydrate polymer in polymerization, relative to a width direction of the endless belt. B (cm$^2$) represents a maximum cross-sectional area of space between the endless belt of the continuous polymerization apparatus and the ceiling plane of the continuous polymerization apparatus, relative to a width direction of the above endless belt.

A production method for obtaining the water-absorbing resin which composes the water-absorbing agent of the present invention, is preferably a continuous production method having an apparatus height ratio, specified by the following formula 2, in the range of 10 to 500.

[Formula 2]

$$\text{[Formula 2]}$$

$$\text{(Belt ratio)} = \text{E/D}$$

In the above formula, D (cm) represents a feeding thickness of the aqueous solution of the monomer. E (cm) represents a maximum height from the endless belt to the ceiling plane of the continuous polymerization apparatus.

(Polymerization initiation temperature)

According to a preferable embodiment, in the polymerization step in producing the water-absorbing resin which composes the particulate water-absorbing agent of the present invention, the polymerization initiation temperature is, in view of enhanced absorption rate, preferably equal to or higher than 40°C, more preferably equal to or higher than 50°C, still more preferably equal to or higher than 60°C, particularly preferably equal to or higher than 70°C, and most preferably equal to or higher than 80°C. On the other hand, the upper limit value of the polymerization initiation temperature is preferably equal to or lower than 150°C, more preferably equal to or lower than 110°C, and still more preferably equal to or lower than 100°C. The too low polymerization initiation temperature would not provide enhancement of absorption rate, while the too high polymerization initiation temperature would decrease other physical properties such as absorbency or extractable content. A high monomer temperature has also an advantage in terms of capable of removing dissolved oxygen easily. Further, the polymerization initiation temperature below 40°C would not provide foaming polymerization, and would raise a problem of not only decreasing the productivity caused by extended induction period and polymerization time but also decreasing the physical properties of the water-absorbing resin.

The polymerization initiation temperature can be observed by white turbidity, viscosity increase or temperature increase of the aqueous solution of the monomer. The polymerization initiation temperature or peak polymerization temperature to be explained later can be measured by using a contact-type temperature sensor such as a general mercury thermometer, an alcohol thermometer, a platinum resistance thermometer sensor, a thermocouple or a thermistor, or a non-contact-type one such as a radiation thermometer. Because induction period in redox polymerization by activated energy rays such as UV rays , an oxidizing agent and a reducing agent, thermally initiated polymerization by an azo-type initiator,
is generally as short as 1 second to 1 minute, the polymerization initiation temperature may be specified as a temperature of the aqueous solution of the monomer before the addition of the initiator, or before irradiation with the activated energy rays.

(Peak temperature)

According to another preferable embodiment, a peak polymerization temperature in the polymerization step in producing the water-absorbing resin which composes the water-absorbing agent of the present invention is specified. The peak polymerization temperature is referred to as a peak achieving temperature of a polymer gel or monomer during progress of polymerization. Specifically, the relevant peak polymerization temperature is, in view of enhancement of absorption rate, preferably equal to or higher than 100°C, more preferably equal to or higher than 105°C, still more preferably equal to or higher than 110°C, particularly preferably equal to or higher than 115°C, and most preferably equal to or higher than 120°C. On the other hand, the upper limit of the peak polymerization temperature is preferably equal to or lower than 150°C, more preferably equal to or lower than 140°C, and still more preferably equal to or lower than 130°C. The too low temperature would not provide enhancement of absorption rate, while the too high temperature may decrease other physical properties such as absorbency or extractable content.

In the measurement of temperature of a polymerization system, a non-contact temperature sensor or an infrared thermometer may be suitably selected.

According to still another preferable embodiment, a difference between the polymerization initiation temperature and the peak polymerization temperature, $\Delta T$, is over 0°C and preferably equal to or lower than 70°C, more preferably equal to or lower than 60°C, further preferably equal to or lower than 50°C, further more preferably equal to or lower than 40°C, particularly preferably equal to or lower than 30°C, and most preferably equal to or lower than 25°C. The $\Delta T$ larger than 70°C may cause decrease in absorption rate of the obtained water-absorbing resin decreases. As an example of a method to obtain heat of polymerization which provides temperature profile during polymerization as mentioned above, for example, it may included to increase concentration of the monomer up to equal to or higher than 30% by weight.

(Polymerization initiator)

A polymerization initiator to be used is not especially limited. Thermally degradable initiators (for example, persulfate: sodium persulfate, potassium persulfate, ammonium persulfate; peroxide: hydrogen peroxide, t-butyl peroxide, methyl ethyl ketone peroxide); an azo compound: an azonitrile compound, an azoamidine compound, a cyclic azoamidine compound, an azoamide compound, an alkylazo compound, 2,2'-azobis(2-amidinopropane) dihydrochloride, 2,2'-azobis[2-(2-imidazoline-2-yl)propane] dihydrochloride); or a photodegradable initiator(for example, a benzoin derivative, a benzil derivative, an acetophenone derivative, a benzophenone derivative or an azo compound) may be used.

In view of decreasing capability of cost or residual monomers, persulfate is preferably used as the polymerization initiator. It is also a preferable method to use a photodegradable initiator and ultraviolet rays in combination. More preferably, a photodegradable initiator and a thermally degradable initiator are used in combination. The amount of the polymerization initiator is usually 0.001 to 1% by mole, more preferably 0.01 to 0.5% by mole, and still more preferably 0.05 to 0.2% by mole, relative to the total amount of the monomers.

(Polymerization time)

A polymerization time is not especially limited. It is preferably equal to or shorter than 10 minutes, more preferably equal to or shorter than 5 minutes, still more preferably equal to or shorter than 3 minutes, particularly preferably equal to or shorter than 2 minutes, and more preferably equal to or shorter than 1 minute. The polymerization time over 10 minutes would not only decrease productivity of the obtained polymer (hydrate polymer, base polymer, and water-absorbing resin) but also may decrease physical properties such as absorption rate.

The polymerization time can be calculated by measuring a period from a time when an aqueous solution of monomer is fed into a polymerization container, and polymerization initiation conditions are arranged (a light irradiation start time when a photodegradable initiator is used; and a time when an aqueous solution of monomer and a polymerization initiator are fed into a polymerization container when no photodegradable initiator is used) to a time showing a peak temperature. To be specific, the polymerization time can be calculated by measurement of (induction period) + (period from polymerization initiation time to reaching to a peak temperature).

(Adjustment method for initiation temperature)

To heat the monomer to a desired temperature (for example, equal to or higher than 40°C), a polymerization container itself may be heated, or an aqueous solution of monomer may be heated in a pipeline when supplied to a polymerization container. A heat by neutralization of a monomer is preferably used for rising temperature. A generated heat by neutralization and/or a heat by hydration is preferably used, because it is utilized effectively not only in temperature rise of an aqueous solution of monomer but also in removal of dissolved oxygen, and it is also capable of enhancing absorption rate.

In this way, to effectively utilize heat by neutralization and/or heat by hydration, it is preferable to carry out neutralization in a heat-insulating state, and it is more preferable to carry out polymerization continuously while carrying out neutralization continuously. To attain this, for example, it is preferable to use a container having heat loss suppressed as much as possible. As the material, a material having its non-contacting parts of resin, rubber, stainless steel covered with an insulating material, may be preferably used.

(Deaeration)

According to a preferable embodiment of the present invention, polymerization is performed while setting an amount of dissolved oxygen to a level preferably equal to or lower than 4 mg/L, more preferably equal to or lower than 2 mg/L, and most preferably equal to or lower than 1 mg/L, relative to total amount of an aqueous solution of monomer.

As a method for setting the amount of dissolved oxygen within the range, an inert gas may be blown in or deaeration under reduced pressure may be performed, before feeding of a polymerization initiator. In such a case, a special apparatus or an operation cost may be required. Therefore, in a preferable embodiment of the present invention, dissolved oxygen may be removed by utilizing heat by neutralization and/or heat by hydration, to increase a temperature of an aqueous solution of monomer and to vaporize dissolved oxygen.

To measure an amount of dissolved oxygen, a measurement apparatus (DO meter UD-1 model, manufactured by Central Kagaku Corp.) may be used, for example. The amount of dissolved oxygen can be measured by sealing an aqueous solution of monomer prepared under nitrogen atmosphere without entraining air bubble, cooling under gentle stirring, and measuring an amount of dissolved oxygen when the solution temperature reaches 50°C.

It is also preferable to further remove oxygen by heat by neutralization, after partial removal of oxygen, in advance, from a part or whole of acrylic acid, an alkali aqueous solution, water as raw materials of an aqueous solution of monomer. In the case where polymerization is initiated at a high temperature of equal to or higher than 80°C, by neutralization of acrylic acid and the alkali in line-mixing, followed by line-mixing of the polymerization initiator, it is preferable to decrease an amount of removed oxygen in advance, or not to remove oxygen, for acrylic acid, an aqueous alkali solution or water, as the raw materials, to prevent polymerization initiation in the pipeline.

(Increase in solid content)

It is preferable that production of the water-absorbing resin which composes the water-absorbing agent of the present invention, is performed under polymerization conditions to increase solid content, in view of obtaining the water-absorbing resin having high absorption rate.

According to a preferable example of a polymerization method, polymerization proceeds while increasing a concentration of solid content, due to rapid increase in temperature of a system after polymerization initiation, which leads to reach a boiling point at low polymerization rate, for example, at a polymerization rate of 10 to 20% by mole relative to total monomer as 100% by mole, and generate steam. The concentration of solid content is increased by effective utilization of heat of polymerization. An amount of increase in solid content during polymerization is preferably equal to or higher than 1% by weight, more preferably equal to or higher than 2% by weight, and still more preferably equal to or higher than 3% by weight.

An increase in solid content is specified as a concentration difference between a solid content of an aqueous solution of monomer and a solid content of the obtained hydrate gel-like polymer. The solid content of a hydrate gel-like polymer to calculate an increase in a solid content is calculated by cutting out a small quantity of a part of a hydrate polymer taken out from a polymerization container, cooling quickly, quickly cutting finely with a pair of scissors, putting 5 g thereof into a Petri dish, and drying in a dryer at 180°C for 24 hours. A concentration of solid content of a particulate hydrate polymer may be calculated as reduced amount in drying, in the case where 5 g of the sample is put into the Petri dish, and drying it in a dryer at 180°C for 24 hours.

As a typical method for increasing a solid content in a polymerization step, for example, in the case of polymerization under normal pressure, there may be included a method for attaining such polymerization conditions that polymerization temperature is already equal to or higher than 100°C at the time when polymerization rate is 40% by mole, and polymerization temperature is also equal to or higher than 100°C even at the time when polymerization rate is 50% by mole. More preferably, there may be included such polymerization conditions that polymerization temperature is already equal to or higher than 100°C at the time when polymerization rate is 30% by mole, and polymerization temperature is also equal to or higher than 100°C even at the time when polymerization rate is 50% by mole. Particularly preferably, there may be included such polymerization conditions that polymerization temperature is already at equal to or higher than 100°C at the time when polymerization rate is 20% by mole, and polymerization temperature is also equal to or higher than 100°C even at the time when polymerization rate is 50% by mole. In the case of polymerization under reduced pressure, for example, such polymerization conditions may be used that polymerization temperature is already boiling point at the time when polymerization rate is 40% by mole, and polymerization temperature is also boiling point even at the time when polymerization rate is 50% by mole. More preferably, such polymerization conditions may be used that polymerization temperature is already boiling point at the time when polymerization rate is 30% by mole, and polymerization temperature is also at boiling point even at the time when polymerization rate is 50% by mole. Particularly preferably, such polymerization conditions may be included that polymerization temperature is already boiling point at the time when polymerization rate is 20% by mole, and polymerization temperature is also boiling point even at the time when polymerization rate is 50% by mole.

As a more preferable method for obtaining the water-absorbing resin which composes the particulate water-absorbing agent of the present invention, in addition to the above, there may be exemplified a method for controlling a polymerization initiation temperature to a temperature equal to or higher than 40°C or a peak polymerization temperature to a temperature equal to or higher than 100°C, preferably a polymerization initiation temperature to 80 to 110°C or a peak polymerization temperature to 100 to 130°C, and for satisfying conditions of the following A or B.

[Method A]

This is a method which comprises using the cross-linking agent in an amount of preferably 0.0070 to 0.020% by mole, more preferably 0.010 to 0.020% by mole, relative to acrylic acid and/or a salt thereof as the monomer, and using in polymerization an aqueous solution of monomer containing the monomer used in performing of polymerization in a concentration (a polymerization concentration, that is, a ratio of monomer solid content in the aqueous solution of the monomer) of 40 to 60% by weight, preferably 40 to 55% by weight, and particularly preferably 45 to 55% by weight.

[Method B]

This is a method which comprises using the cross-linking agent in an amount of preferably 0.0030 to 0.010% by mole, and more preferably 0.0050 to 0.009% by mole, relative to acrylic acid and/or a salt thereof as the monomer, and using in polymerization an aqueous solution of monomer containing the monomer used in performing of polymerization in a concentration (a polymerization concentration, that is, a ratio of monomer solid content in the aqueous solution of the monomer) of 60 to 80% by weight, preferably 65 to 80% by weight, and particularly preferably 65 to 70% by weight.

(Drying)

In the case where a polymer obtained by polymerization is gel-like, the gel-like polymer may be dried preferably at 70 to 250°C, more preferably at 120 to 230°C, and crushed, if necessary. A powder of the water-absorbing resin having a weight average particle diameter (D50) of 10 to 1000 $\mu$m can be obtained. Although a drying method is not especially limited, a drying method for subjecting a material under moving to good contacting with hot air or a heat conduction surface, such as a stirring drying method, a fluid-bed drying methods or an air-flow drying method is preferably used.

In the case where the hydrate, gel-like polymer formed by aqueous solution polymerization of the monomer component, which will become the water-absorbing resin by polymerization, has thick plate-like, block-like, sheet-like shape or the like, which is difficult to dry as it is, a gel may be crushed to a particle diameter of usually equal to or smaller than 10 mm, more preferably equal to or smaller than 3 mm, and then subjected to drying. In gel crushing, water or a surfactant, a water-soluble polymer may be added in an amount of preferably 0 to 30% by weight, more preferably 0 to 10% by weight (relative to the water-absorbing resin), to enhance gel crushing performance and physical property.

Further, a water-absorbing resin may be optionally obtained via each step of crushing, classification or surface-treatment after drying. It is preferable that a fine powder or a coarse particle obtained in such a classification step is applied to polymerization as a solid, in the present invention.

(5) Gel fine crushing step

The hydrate gel-like polymer obtained by polymerization may be subjected to drying as it is. In the case of aqueous solution polymerization, however, it is subjected to drying after fine crushing using a gel crusher, if necessary. Shape of the water-absorbing resin to be used in the present invention is not especially limited, and may be arbitrarily selected form such as granule-like, powder-like, flake-like, fiber-like shape, for example.

Therefore, fine crushing may be performed by various methods. For example, a method for crushing by extruding from a screw-type extruder having a porous structure with arbitrary shape may be exemplified.

(6) Drying step

A drying temperature is not especially limited. It may be in the range of 50 to 300°C (in the case of equal to or lower than 100°C, it is preferable to perform drying by azeotropic dehydration or under reduced pressure), and preferably 100 to 250°C, and still more preferably 150 to 200°C, to enhance absorbency.

As a drying method, various methods may be adopted, such as heating drying, hot air drying, reduced pressure drying, fluid-bed drying, infrared drying, microwave drying, drum-dryer drying, dehydration by an azeoptrope with a hydrophobic organic solvent, high humidity drying using high temperature steam. According a preferable embodiment, contact drying with a gas having a dew point of 40 to 100°C, more preferably a dew point of 50 to 90°C, can be exemplified.

The hydrate gel-like cross-linked polymer obtained during a polymerization reaction or after completion of the polymerization reaction, by aqueous solution polymerization, may be crushed to give a piece of 0.1 to 50 mm, preferably 0.2 to 10 mm, and more preferably 0.5 to 5 mm, by a predetermined method, and then to drying. A drying temperature is not especially limited. It may be preferably 100 to 250°C, and more preferably 120 to 200°C, for example. In addition, a drying time can be determined as appropriate, and is not especially limited. It may be 10 seconds to 5 hours, and preferably 1 minute to 2 hours.

(7) Pulverizing or classification step

The water-absorbing resin particle obtained by drying may be subjected to a step such as of pulverizing, classification, to control a particle diameter, if necessary, depending on the objective thereof. These methods are described, for example, in WO 2004/69915 Pamphlet. A preferable particle size before surface cross-linking may be controlled in advance within the range of the water-absorbing agent to be described later.

(8) Surface cross-linking

The water-absorbing resin to be used in the present invention may be obtained by subjecting the above water-absorbing resin particle to surface cross-linking processing with a surface cross-linking agent.

As a suitable surface cross-linking agent, there may be used one or two or more members selected among oxazoline compounds (US-B-6,297,319), vinyl ether compounds (US-B-6,372,852), epoxy compounds (US-B-625,488), oxetane compounds (US-B-6,809,158), polyvalent alcohol compounds (US-B-4,734,478), polyamide polyamine-epihalo adducts (US-B-4,755,562 and US-B-4,824,901), hydroxyacrylamide compounds (US-B-6,239,230), oxazolidinone compounds (US-B-6,559,239), bis- or poly-oxazolidinone compounds (US-B-6,472,478), 2-oxotetrahydro-1,3-oxazolidine compounds (US-B-6,657,015), alkylene carbonate compounds (US-B-5,672,633) . In addition, a water-soluble cation such as of an aluminum salt (US-B-6,605,673 and US-B-6,620,899) may be used in combination with the surface cross-linking agent. Also, an alkali metal salt (US-A-2004-106,745), an organic acid or an inorganic acid (US-B-5,610,208). may be used in combination. In addition, polymerization of a monomer on the surface of the water-absorbing resin can be performed as the surface cross-linking (US-A-2005-48,221). Among these, a polyvalent alcohol compound, a polyvalent epoxy compound, a polyvalent amine compound or a salt thereof, an alkylene carbonate compound are preferably used. Since they generally impart hydrophilic property to the surface, the production method for the above water-absorbing resin can be applied effectively.

In the present invention, it is preferable that a hydrophilic organic compound such as a polyvalent alcohol compound is contained on the surface of the water-absorbing resin. More preferably, the hydrophilic organic compound is contained in an amount of 0.001 to 10 parts by weight, and still more preferably 0.01 to 5 parts by weight, relative to 100 parts by weight of a precursor of the water-absorbing resin. As used herein, the hydrophilic organic compound is referred to as a compound which dissolves in an amount of equal to or more than 1 g, preferably equal to or more than 10 g, into 100 g of water at normal temperature.

As a specific surface cross-linking agent, there may be included polyvalent alcohol compounds such as (di, tri, tetra, poly)ethylene glycol, (di, poly)propylene glycol, 1,3-propanediol, 2,2,4-trimethyl-1,3-petanediol, (poly)glycerin, 2-butene-1,4-diol, 1,4-butanediol, 1,3-butanediol, 1,5-pentanediol, 1,6-hexanediol, trimethylolpropane, (di, tri) ethanol amine, pentaerythritol, sorbitol; epoxy compounds such as (poly)ethylene glycol diglycidyl ether, (di, poly) glycerol polyglycidyl ether, (di, poly)propylene glycol diglycidyl ether, glycidol; polyvalent oxazoline compounds such as 1,2-ethylenebisoxazoline; alkylene carbonate compounds such as 1,3-dioxolane-2-one; a polyvalent metal compounds such as aluminum sulfate.

The surface cross-linking agent is preferably used in an amount of 0.01 to 10 parts by weight, and more preferably 0.5 to 5 parts by weight, relative to 100 parts by weight of a precursor of the water-absorbing resin. The amount of the surface cross-linking agent below 0.01 parts by weight may decrease liquid permeability. The use mount of over 10 parts by weight may extremely decrease absorbency.

(9) Solvent

In the case where the water-absorbing resin is mixed with the surface cross-linking agent, it is preferable to use water as a solvent. In the case where total amount of water is 1 to 10 parts by weight relative to 100 parts by weight of the water-absorbing resin, an aqueous solution of the surface cross-linking agent can penetrate sufficiently in the surface of the water-absorbing resin, and may form a multilayer of surface cross-linked layers having suitable thickness and density.

In the case where the water-absorbing resin is mixed with the surface cross-linking agent, a hydrophilic organic solvent may be used as the solvent, if necessary. As the hydrophilic organic solvent, for example, lower alcohols such as methyl alcohol, ethyl alcohol, n-propyl alcohol, isopropyl alcohol, n-butyl alcohol, isobutyl alcohol, t-butyl alcohol; ketones such as acetone; ethers such as dioxane, tetrahydrofuran, alkoxypolyethylene glycol; amides such as N,N-dimethylformamide; sulfoxides such as dimethylsulfoxide, may be included. The use amount of the hydrophilic organic solvent is preferably equal to or less than 20 parts by weight, and more preferably 0.1 to 10 parts by weight, relative to 100 parts by weight of solid content of the water-absorbing resin, although it depends on kind or particle diameter of the water-absorbing resin.

A method for mixing the water-absorbing resin with the surface cross-linking agent is not especially limited. A method for mixing by directly spraying or dropping a solution of the surface cross-linking agent in water and/or the hydrophilic organic solvent, to the water-absorbing resin, may be preferably used.

A mixing apparatus to be used in mixing the water-absorbing resin with the surface cross-linking agent may be desirably equipped with a large mixing force so as to mix both uniformly and surely. As the mixing apparatus, for example, cylinder-type mixer, double-walled conical-type mixer, V-shape mixer, ribbon type mixer, screw type mixer, rotary disc-type mixer with a flow-type furnace, airflow type mixer, double-arm type kneader, internal mixer, crushing-type kneader, rotation-type mixer, screw-type extruder, turbulizer is suitable.

In order to subject a mixture of the water-absorbing resin and the surface cross-linking agent to cross-linking reaction, a heating processing may be performed. A heating temperature may be selected, as appropriate. The temperature of a heating medium is in the range of preferably 150 to 250°C, and more preferably 180 to 210°C. A heating time is preferably 1 minute to 2 hours. As a suitable example of a combination of the heating temperature and the heating time, the heating temperature may be 180°C for 0.1 to 1.5 hours and the heating time may be at 200°C for 0.1 to 1 hour, for example.

(10) Chelating agent

To obtain the water-absorbing agent of the present invention, it is preferable to add a chelating agent to the water-absorbing resin. By the addition of the chelating agent, the water-absorbing agent, excellent in resistance to urine deterioration or resistance to color protection, can be obtained.

In the present invention, use amount of the chelating agent, in particular, an amino polyvalent carboxylic acid, is a trace amount component, usually 0.00001 to 10 parts by weight, preferably 0.0001 to 1 part by weight, and still more preferably 0.002 to 0.1 parts by weight, relative to 100 parts by weight of the water-absorbing resin as a major component. The use amount of over 10 parts by weight would raise a problem of decrease in absorption amount or the like, as well as it would not provide comparable effects by this use and thus would not be economical. The use amount of lower than 0.00001 parts by weight would not provide sufficient addition effects.

As the chelating agent to be used, a polymer or a non-polymer chelating agent, preferably the non-polymer chelating agent (for example, having a molecular weight or a weight average molecular weight of 40 to 2000, still more 60 to 1000, and in particular 100 to 500) may be used to the monomer or the polymer thereof. As the preferable chelating agent, an aminocarboxylic acid (salt) may be included. The number of carboxyl groups therein may be in the range of 2 to 20, still more 4 to 10, and in particular, 5 to 8.

For example, the aminocarboxylic acid-type chelating agent may include iminodiacetic acid, hydroxyethyliminodiacetic acid, nitrilotriacetic acid, nitrilotrispropionic acid, ethylenediamine tetraacetic acid, hydroxyethylenediamine triacetic acid, hexamethylenediamine tetraacetic acid, diethylenetriamine pentaacetic acid, triethylenetetraamine hexaacetic acid, trans-1,2-diaminocyclohexane tetraacetic acid, bis(2-hydroxyethyl)glycine, diaminopropanol tetraacetic acid, ethylenediamine-2-propionic acid, glycol ether diamine tetraacetic acid, bis(2-hydroxybenzyl)ethylenediamine diacetic acid, and salts thereof.

The phosphoric acid-type chelating agent may include phosphorus compounds such as ethylene diamine-N, N'-di(methylenephosphinic acid), ethylene diamine tetra(methylenephosphinic acid), nitriloacetic acid-di(methylenephosphinic acid), nitrilodiacetic acid-(methylenephosphinic acid), nitriloacetic acid-$\beta$-propionic acid-methylenephosphonic acid, nitrilo tris(methylenephosphonic acid), cyclohexanediamine tetra(methylenephosphonic acid), ethylenediamine-N,N'-diacetic acid-N,N'-di(methylenephosphonic acid), ethylenediamine-N,N'-di(methylenephosphonic acid), ethylenediamine tetra(methylenephosphonic acid), polymethylenediamine tetra(methylenephosphonic acid), diethylenetriamine penta(methylenephosphonic acid), 1-hydroxyethylidene diphosphonic acid, and salts thereof. The compound may be preferably added as an aqueous solution before, during or after the polymerization step, or added during agglomeration.

(11) Agglomeration

In the present invention, it is preferable that the water-absorbing agent is obtained by agglomeration with water or an aqueous solution. In the agglomeration, amount of water or an aqueous solution to be used is usually in the range of 0.5 to 20 parts by weight, and preferably 0.5 to 10 parts by weight, relative to 100 parts by weight of solid content of the water-absorbing resin, although it depends on water content of the water-absorbing resin to be used. Amount of the hydrophilic organic solvent is in the range of 0 to 10 parts by weight, preferably 0 to 5 parts by weight, and more preferably 0 to 3 parts by weight, relative to the water-absorbing resin mixture. Temperature in the addition of the hydrophilic solvent is, in view of mixing performance, in the range of preferably 0 to 80°C, and more preferably 40 to 70°C. In addition, a method of spraying or dropping the hydrophilic solvent onto the water-absorbing resin mixture is preferable, and a method of spraying is more preferable. Size of a droplet to be sprayed is preferably 1 to 300 $\mu$m, and more preferably 1 to 200 $\mu$m.

Heating time is preferably in the range of 1 minute to 2 hours. The addition of water and heating may be performed with the same apparatus, or may be performed with different apparatuses. Stirring may be performed or standing still (no stirring) may be adopted, as long as temperature and water content can be controlled in the heating. It is preferable to harden (to form a weak block state) under standing still (no stirring). It is preferable to harden by laminating the water-absorbing resin added with water so as to give a thickness of preferably 1 to 100 cm, more preferably 5 to 80 cm, and in particular, about 10 to 70 cm, and heating. The water-absorbing resin thus hardened may be then crushed, and preferably classified, to obtain the target water-absorbing agent of the present invention. In this way, the water-absorbing agent of the present invention can be obtained.

(12) Water-absorbing agent of the present invention

The particulate water-absorbing agent comprising a polyacrylate salt-type water-absorbing resin as a main component, provided by the present invention, is characterized by satisfying the following (a) and (b):

(a) absorption capacity without load in artificial urine (A) is equal to or higher than 62 g/g; and
(b) an increase rate of absorption capacity without load in artificial urine (B) relative to artificial urine (A) is equal to or larger than 20%, wherein the polyacrylate salt-type water-absorbing resin is a cross-linked polymer containing acrylic acid (salt) in the repeating units thereof (excluding a cross-linking agent) in an amount of 70 to 100% by mole, and the acrylic acid (salt) as the repeating unit is neutralized in the range of 20 to 100% by mole in the form of an alkali metal salt.

The water-absorbing agent which satisfies the above specific parameters, when actually absorbed with urine, is insusceptible to different kinds of urine, and thus a conventional problem that desired absorption performance cannot be obtained depending on kind of urine, can be avoided.

A mechanism to express such effect is not certain. It, however, is estimated that high molecular weight and narrow molecular weight distribution of the main chain of the water-absorbing resin can form an ideal network. In this regard, however, it is not restricted to this mechanism.

The water-absorbing agent of the present invention has (a) an absorption capacity without load to artificial urine (A) of equal to or higher than 62 g/g, preferably equal to or higher than 64 g/g, and particularly preferably equal to or higher than 66 g/g. The absorption capacity without load to artificial urine (A) below this value would provide too low absorbency and increase use amount of the water-absorbing agent, which would be uneconomical. As for the upper limit, the higher value is the better. In view of easiness in production, an absorption capacity without load of equal to or lower than 100 g/g is preferable and equal to or lower than 90 g/g is more preferable.

In addition, (b) an increase rate of absorption capacity without load in artificial urine (B) relative to artificial urine (A), of the water-absorbing agent of the present invention, is equal to or higher than 20%, preferably equal to or higher than 22%, more preferably equal to or higher than 24%, and still more preferably equal to or higher than 26%. The case outside this range would generate a problem that, in the case of practical absorption of urine, desired absorption performance cannot be obtained depending on kind of urine. The upper limit value is not especially limited. In view of easiness of production, the increase rate of absorption capacity without load in artificial urine (B) relative to artificial urine (A) is equal to or lower than 60%.

Preferably the particulate water-absorbing agent provided by the present invention is characterized by further satisfying the following (d):

> (d) weight average molecular weight (Mw) of the main chain of the water-absorbing resin is equal to or higher than 1,000,000 and molecular weight distribution (Mw/Mn) thereof is equal to or smaller than 3.3.

The water-absorbing agent, which has the above structure and satisfies specified absorbency, can manifest excellent absorbency to any of a plurality of artificial urines with different ion strengths and dissolved components. Specifically, it is possible to obtain a high performance water-absorbing agent having an increase rate of absorption capacity without load in artificial urine (B) relative to artificial urine (A) is equal to or larger than 20%, which will be described in detail in Examples below. By using this water-absorbing agent to an absorbing goods, it is insusceptible to different kinds of urine, when actually absorbed with urine, and thus a conventional problem that desired absorption performance cannot be obtained depending on kind of urine, can be avoided.

The water-absorbing agent of the present invention has (d) the weight average molecular weight (Mw) of the main chain of the water-absorbing resin of equal to or higher than 1,000,000 and molecular weight distribution (Mw/Mn) thereof of equal to or smaller than 3.3. Preferably, the weight average molecular weight (Mw) is equal to or higher than 1,100,000 and the molecular weight distribution (Mw/Mn) thereof is equal to or smaller than 3.0. More preferably, the weight average molecular weight (Mw) is equal to or higher than 1, 200, 000 and the molecular weight distribution (Mw/Mn) thereof is equal to or smaller than 2.8. Still more preferably, the weight average molecular weight (Mw) is equal to or higher than 1, 300, 000 and the molecular weight distribution (Mw/Mn) thereof is equal to or smaller than 2.6. Particularly preferably, the weight average molecular weight (Mw) is equal to or higher than 1,400,000 and the molecular weight distribution (Mw/Mn) thereof is equal to or smaller than 2.4. Use of the water-absorbing agent out of the parameter to the absorbing goods may not attain desired absorption performance, caused by difference of urine kinds. The upper limit of the weight average molecular weight (Mw) is not especially limited. In view of production easiness, it is equal to or lower than 3,000,000. The lower limit value of the molecular weight distribution (Mw/Mn) is also not especially limited. In view of easiness of production, it is equal to or larger than 1.5. The weight average molecular weight (Mw) and value of molecular weight distribution (Mw/Mn) of the main chain are referred to as values obtained by a method to be described in Examples below, respectively.

The water-absorbing agent according to the present invention contains particles having a particle diameter of equal to or larger than 300 $\mu$m and smaller than 600 $\mu$m in an amount of preferably equal to or larger than 50% by weight, more preferably equal to or larger than 55% by weight, still more preferably equal to or larger than 60% by weight, and most preferably equal to or larger than 65% by weight. The upper limit is 100% by weight. The amount out of this range would not be preferable due to deterioration of hand feeling of the absorbing goods, or risk of dust generation.

(13) Shape of the water-absorbing resin

The water-absorbing agent of the present invention is particulate, in view of water-absorption characteristics. A weight average particle diameter (D50) is preferably 200 to 500 $\mu$m, more preferably 200 to 450 $\mu$m, and still more preferably 250 to 400 $\mu$m. In addition, the lower content of particles having a particle diameter smaller than 150 $\mu$m as determined by a JIS standard sieve is the better. The amount of particles having a particle diameter smaller than 150 $\mu$m as determined by a JIS standard sieve is usually 0 to 5% by weight, preferably 0 to 3% by weight, and particularly preferably 0 to 1% by weight. Similarly, the lower content of particles having a particle diameter of equal to or larger than 850 $\mu$m JIS standard sieve is the better. The amount of particles having a particle diameter of equal to or larger than 850 $\mu$m JIS standard sieve is usually 0 to 5% by weight, preferably 0 to 3% by weight, and particularly preferably 0 to 1% by weight. In the water-absorbing agent of the present invention, particles having a particle diameter of 300 to 600

μm are contained in an amount of equal to or more than a certain level. Specifically, the amount of the particles having particle diameter within such a range is preferably 50 to 100% by weight, more preferably 55 to 100% by weight, particularly preferably 60 to 100% by weight, and most preferably 65 to 100% by weight, relative to total amount of the particles. Logarithmic standard deviation ($\sigma\zeta$) of particle size distribution of the water-absorbing agent of the present invention is preferably 0.20 to 0.45, more preferably 0.25 to 0.40, and particularly preferably 0.28 to 0.35. The particle size is measured with a standard sieve classification (JIS Z8801-1(2000), or an equivalent product thereof), according to the description of US-A-2005-118423. If the weight average particle diameter, ratio of particles having a diameter below 150 μm or particles having a diameter of equal to or larger than 850 μm, ratio of particles having a diameter of 300 to 600 μm, and logarithmic standard deviation ($\sigma\zeta$) of particle size distribution are outside the above ranges, hand feeling would be deteriorated, or risk of dust generation would increase. In particular, increase in particles having a diameter of equal to or larger than 850 μm, would cause deterioration of hand feeling significantly, or decrease in absorption rate, any of which would not be preferable. Increase in particles having a diameter of below 150 μm would not only increase risk of incurring dust, but also form lumpy flour in absorption of water, which would make uniform absorption difficult, resulting in decrease in absorption rate, any of which would not be preferable.

A bulk specific gravity (specified in the description of US-B-6,562,879) of the water-absorbing agent of the present invention is usually 0.30 to 0.90, preferably 0.60 to 0.80, and more preferably 0.65 to 0.75. In the case where the bulk specific gravity is outside the above range, desired liquid permeability may not be obtained, or property could be decreased in receiving impact, any of which would not be preferable.

Shape of the particulate water-absorbing agent is not especially limited, and may include sphere-like, nearly sphere-like, irregularly crushed-type (crushed substance), bar-like, polyhedron-like, sausage-like (for example in the description of US-B-4, 973, 632), particles with wrinkles (for example, in the description of US-B-5,744,564). These particles may be single particles, agglomerated particles, or a mixture thereof. In addition, these particles may be porous by foaming. Preferable shape of these particles includes, in view of handling in production, or production cost, irregularly crushed-type single particles or agglomerated substances thereof.

(14) Other physical properties of the water-absorbing agent

The water-absorbing agent of the present invention preferably has an extractable content of 20 to 70% by weight, more preferably 25 to 65% by weight, and still more preferably 30 to 60% by weight. If the extractable content exceeds the upper limit, there may be risk to raise a problem that gel shape is not able to be maintained in water absorption. If the extractable content is less than lower limit, the production steps of the water-absorbing agent would become complicated and cost would increase, any of which would not be preferable. The extractable content is obtained by a method to be described in Examples below.

(15) Additive

The water-absorbing agent of the present invention preferably contains water. The content of water (as reduced to as a weight in drying at 180°C for 3 hours) is preferably 0.1 to 15% by weight, more preferably 1 to 10% by weight, and particularly preferably 2 to 6% by weight, relative to total amount of the water-absorbing agent. The low content of water would make water-absorption rate and impact resistance inferior, while the high water content would decrease absorbency. Preferably, a water-insoluble inorganic powder and a chelating agent are contained in amounts within the above ranges, in view of enhancing durability (prevention of urine deterioration) and expressing effects of preventing coloration.

If necessary, a deodorant, an antibacterial agent, a fragrance, a surfactant, a fertilizer, an oxidizing agent, a reducing agent, a hydrophilic polymer, a hydrophobic polymer such as paraffin, a thermoelastic resin such as polyethylene, polypropylene may be contained in an amount of usually 0 to 20% by weight, preferable 0.001 to 10% by weight, relative to total amount of the water-absorbing agent.

(16) Water-absorbing goods

Applications of the water-absorbing agent of the present invention are not especially limited. Preferably, the water-absorbing agent can be applied to an absorbing body and an absorbing goods such as a diaper, a pet sheet, an incontinence pad, a sanitary napkin.

The absorbing body can be obtained by using the water-absorbing agent. As used herein, the absorbing body is referred to as an absorbing material formed by the water-absorbing agent and hydrophilic fibers as main components. A content (core concentration) of the water-absorbing agent is preferably 1 to 50% by weight, more preferably 5 to 40% by weight, and particularly preferably 5 to 30% by weight, relative to total weight of the water-absorbing agent and the hydrophilic fibers.

The absorbing goods of the present invention may be provided with the absorbing body, a front sheet having liquid permeability and a back sheet having liquid non-permeability. The absorbing goods of the present invention is capable of quickly absorbing a large quantity of aqueous liquid added all at once, while the aqueous liquid does not go back. It is excellent in feeling during the use thereof due to its small re-wet and good dry-feeling.

Examples

Explanation will be given below specifically on Examples and Comparative Examples of the present invention. However, the present invention should not be limited to these Examples. In these Examples, physical property and performance of the water-absorbing resin, water-absorbing agent and absorbing goods were measured by the following methods. In addition, physical property of the water-absorbing agent and water-absorbing resin were measured similarly.

(Measurement condition)

25°C ± 2°C, relative humidity 50% RH

(Measurement liquid)

Artificial urine (A) : An aqueous 0.90% by weight sodium chloride solution (physiological saline)
Artificial urine (B) : A uniform and transparent solution obtained by mixing 0.25 g of calcium chloride dihydrate, 2. 0 g of potassium chloride, 0.50 g of magnesium chloride hexahydrate, 2.0 g of sodium sulfate, 0.85 g of ammonium dihydrogen phosphate, 0.15 g of diammonium hydrogen phosphate, and 994.25 g of pure water.
In the case where the water-absorbing agent in a diaper is in a wetted state, measurement is performed after drying till water content of the water-absorbing agent becomes equilibrium (around 5% by weight, 2 to 8% by weight), by drying under reduced pressure, as appropriate (for example, at 60 to 80°C for about 16 hours).

(1) Absorption capacity without load in artificial urine (A)

A water-absorbing agent passing through a 600 $\mu$m sieve but not passing through a 300 $\mu$m sieve was selected to use as a sample for measurement. Into a bag made of nonwoven fabric ("Heatron Paper GS-22" (85 mm × 60 mm) , manufactured by Nangoku Pulp Industry Co., Ltd.), 0.20 g of the water-absorbing agent was uniformly charged, and after sealing, the bag was immersed in an artificial urine (A) for 30 minutes. Then, the bag was pulled up and drained off the water by using a centrifugal separator (a centrifugal separator: model H-122, manufactured by Kokusan Co., Ltd.) under a centrifugal force (250 G) described in EDANA Absorbency III441.1-99, for 3 minutes to weigh the bag, W1 (g). A similar process was performed without using the water-absorbing agent to weigh the bag, W0 (g). By using the weights, W1 and W0, absorption capacity without load (g/g) in the artificial urine (A) was calculated according to the following formula 3.
```
[Formula 3]
Absorption capacity without load in artificial urine (A),
(GV(A))  (g/g)  =  {(W1  (g)  -  W0  (g))  /  (weight  of
water-absorbing agent (g))} - 1
```

(2) Absorbency under non-pressure to artificial urine (B)

A water-absorbing agent passing through a 600 $\mu$m sieve but not passing through a 300 $\mu$m sieve was selected to use as a sample for measurement. Into a bag made of nonwoven fabric ("Heatron Paper GS-22" (85 mm × 60 mm), manufactured by Nangoku Pulp Industry Co., Ltd.), 0.20 g of the water-absorbing agent was uniformly charged, and after sealing, the bag was immersed in an artificial urine (B) for 30 minutes. Then, the bag was pulled up and drained off the water by using a centrifugal separator (a centrifugal separator: model H-122, manufactured by Kokusan Co., Ltd.) under a centrifugal force (250 G) described in EDANA Absorbency III441.1-99, for 3 minutes to weigh the bag, W3 (g). A similar process was performed without using the water-absorbing agent to weigh the bag, W2 (g). By using the weights, W3 and W2, absorption capacity without load (g/g) in the artificial urine (B) was calculated according to the following formula 4.

[Formula 4]

Absorption capacity without load to artificial urine (B),

(GV(B)) (g/g) = {(W3 (g) - W2 (g)) / (weight of

water-absorbing agent (g))} - 1

In addition, from the values obtained from the formula 3 and formula 4, an increase rate of absorption capacity without load in artificial urine (B) relative to artificial urine (A) was calculated according to the following formula 5.

[Formula 5]

Increase rate of absorption capacity without load in artificial urine (B) relative to artificial urine (A) [%]

= 100 × {(absorption capacity without load in artificial

urine (B) (g/g)) - (absorption capacity without load in

artificial urine (A) (g/g))} / (absorption capacity without

load in artificial urine (A) (g/g))

(3) Extractable content

An extractable content was measured according to a method described in WO 2005/092,956 Pamphlet. Specifically, 184.3 g of physiological saline was weighed into a 250-ml container ("Pack Ace", manufactured by Teraoka Corp.). Into the aqueous solution, 1.00 g of a water-absorbing agent passing through a 600 $\mu$m sieve but not passing through a 300 $\mu$m sieve, which is selected as a sample water-absorbing agent, was added. The mixture was stirred at 600 rpm for 16 hours using a stirrer chip (length 35 mm, diameter 7 mm, bar-like shape) made of Teflon (registered trade name), to extract water-extractables in the water-resin of the water-absorbing agent. This extracted solution was filtered through a filter paper (product name: (JIS P3801, No. 2) having a thickness of 0.26 mm, and a retention particle diameter of 5 $\mu$m, manufactured by ADVANTEC Toyo Ltd.), to obtain a filtrate. 50.0 g of the filtrate was weighed to use as a solution for measurement.

First, physiological saline alone was titrated with an aqueous 0.1 N NaOH solution so as to give pH level of 10, and subsequently titrated with an aqueous 0.1 N HCl solution so as to give pH level of 2.7, to obtain blank titers ([bNaOH] (ml) and [bHCl] (ml)). By performing the similar titrating operation on the solution for measurement, the titers ([NaOH] (ml) and [HCl] (ml)) were determined.

For example, in the case of the water-absorbing resin, which is composed of known amounts of acrylic acid and a sodium salt thereof, water-extractable content in the water-absorbing agent can be calculated in accordance with the following formula 6, based on the average molecular weight of the monomer and the titer obtained from the above operation. When the amounts are unknown, the average molecular weight of the monomer can be calculated by using neutralization ratio determined by the titration.

[Formula 6]

    Extractable content (% by weight)

    = 0.1 × (average molecular weight) × 184.3 × 100 × ([HCl]
    - [bHCl]) / 1000 / 1.0 / 50.0


    Neutralization ratio (% by mole)

    = [1 - ([NaOH]-[bNaOH]) / ([HCl]-[bHCl])] × 100

(4) Weight average molecular weight (Mw) and molecular weight distribution (Mw/Mn) of main chain of water-absorbing resin

The measurement is for measuring a molecular weight of a polymer, after removing by hydrolysis a cross-linking derived from a cross-linking agent in the water-absorbing resin.

First, a water-absorbing agent passing through a 600 $\mu$m sieve but not passing through a 300 $\mu$m sieve was selected to use as a sample for measurement. In the case where particles with a particle diameter of 300 to 600 $\mu$m are not present in all of the particles of the water-absorbing agent, or in the case where they are included in an amount only below 30% by weight in all of the particles, the measurement shall be substituted with measurement by using all of the particles. Into a 20-ml sealable glass bottle, 0.04 g of the water-absorbing agent and 10 mL of an aqueous 0.1 N sodium hydroxide solution were charged and sealed, left to stand for one day in a thermostatic oven at 60°C, and further left to stand at room temperature for 9 days, to hydrolyze a cross-linking of the water-absorbing resin to be solubilized (in the case where it is impossible to solubilize under these conditions, a cycle of leaving to stand at 60°C for one day, and leaving to stand at room temperature for 9 days is repeated, till attaining solubilization). By mixing 1.0 ml of the obtained solution with 9.0 ml of an eluent which is described later, for dilution, and filtering through a 0.45 $\mu$m filter (product name: Chromatodisk, 25A, an aqueous system, 0.45 $\mu$m, manufactured by GL Science Co., Ltd.), a sample for GPC measurement was obtained.

GPC measurement conditions:

An eluting solution: An aqueous solution obtained by dissolving $NaH_2PO_4 \cdot 2H_2O$ and $Na_2HPO_4 \cdot 12H_2O$ in ultra pure water (with a specific resistance of equal to or higher than 15 M$\Omega$-cm) so as to give a concentration of 60 mM and 20 mM, respectively.

Standard samples: Powder of "PolyAcrylicAcid Standard", purchased from American Polymer Standard Co., Ltd. (A: Mw=1,930, Mn=1,230, B: Mw =3,800, Mn=2,280, C: Mw=8,300, Mn=6,200, D:Mw=18,100, Mn=12, 800, E: Mw=36, 900, Mn=23,100, F: Mw =70,900, Mn=39,400, G: Mw=131,200, Mn=78,400, H: Mw=440,000, Mn=251,000, K: Mw=695,100, Mn=493,000, L: Mw=958,000, Mn=638,700, N: Mw=1,360,000, Mn=888,900, 0: Mw=1,700,000, Mn=1,100,000) (A calibration curve was prepared by dissolving 0.004 g of the powder into 10 ml of the eluent, and measuring the solution by GPC).

GPC system: SHODEX GPC-SYSTEM-21

Guard Column: SHODEX Asahipak GF-1g7B (manufactured by Showa Denko K. K.)

Sample Column: Two sets of TOSOH GMPWXL were connected in series (manufactured by Tohso Co., Ltd.)

Charging rate: 0.5 ml/min

Detector: 205 nm

Evaluation items: Weight average molecular weight (Mw), number average molecular weight (Mn), molecular weight distribution =Mw/Mn

(5) Weight average particle diameter (D50) and logarithmic standard deviation ($\sigma\zeta$)

A particle size distribution and logarithmic standard deviation thereof were determined by classification of 10 g of a water-absorbing agent with JIS standard sieves, according to WO 2005/09,956 Pamphlet. To be specific, by classifying the water-absorbing agent with JIS standard sieves with a mesh opening of 850 $\mu$m, 710 $\mu$m, 600 $\mu$m, 500 $\mu$m, 425 $\mu$m, 300 $\mu$m, 212 $\mu$m, 150 $\mu$m, 106 $\mu$m, residual percentages R' s were plotted in logarithmic probability paper. A weight average particle diameter (D50) was read. In addition, logarithmic standard deviation ($\sigma\zeta$) of particle size distribution was calculated according to the following formula 7.

[Formula 7]

$$\sigma\zeta = 0.5 \times \ln (X2 / X1)$$

(wherein X1 and X2 represent a particle diameter when R=84.1% by weight and R=15.9% by weight, respectively.)

[Example 1]

(Polymerization)

In a 1 liter container made of a polypropylene resin, 289.39 g of acrylic acid, 0.319 g (0.0152% by mole, relative to the monomer) of polyethylene glycol diacrylate (average adduct mole number of ethylene oxide: 9, average molecular weight: 522), as an internal cross-linking agent, 1.8 g of a 1% by weight aqueous solution of diethylenetriaminepentaacetic acid pantasodium salt as a chelating agent, and 3.6 g of a 1.0% by weight acrylic acid solution of IRGACURE (registered tradename) 184 as a polymerization initiator, were mixed, to prepare a solution (A). Separately, 254.09 g of an aqueous 48.5% by weight sodium hydroxide solution and 241.82 g of ion exchanged water with temperature adjusted at 50°C, were mixed, to prepare a solution (B). By mixing the solution (B) quickly into the solution (A) under stirring at 500 rpm using a magnetic stirrer chip with a length of 50 mm, an aqueous solution of a monomer (C) was obtained. A temperature of the aqueous solution of the monomer (C) increased up to 102°C, by heat of neutralization and heat of dissolution.
Then, at the time when the temperature of the aqueous solution of the monomer (C) decreased to 97 °C, 11 g of an aqueous 3% by weight sodium persulfate solution was added to the aqueous solution of the monomer (C). Soon after stirring for about 1 second, the solution was poured, in an open system, into a stainless steel vat-type container equipped with a liner of Teflon (registered trade name) on the inner face, and having the surface heated with a hot plate (NEO HOTPLATE H1-1000, manufactured by IUCHI SEIEIDO Co., Ltd.) set at 130°C. The stainless steel vat-type container has a size of the bottom face 250 mm×250 mm, the upper face 640 mmx640 mm, and height 50 mm, a trapezoid central cross-section, and the upper face thereof was open. At the same time of pouring of the aqueous solution of the monomer into the stainless steel vat-type container, UV rays were irradiated from a UV rays irradiation apparatus (TOSCURE 401, model name: HC-04131-B, lamp: H400L/2, manufactured by Rison Toshiba Lighting Co., Ltd.), installed at a height of 600 mm from the bottom of the stainless steel vat-type container.
Polymerization was initiated soon after pouring of the aqueous solution of the monomer into the vat, and cast polymerization in the aqueous solution proceeded under generation of steam. Polymerization temperature reached a peak temperature within 1 minute. After three minutes from irradiation start, irradiation of UV rays was stopped to take out a hydrate polymer (hydrate gel). These operations were performed in an open air system.

(Gel fine crushing)

After taking out the hydrate gel-like cross-linked polymer and cutting it with a pair of scissors to rectangular shape with a width of 30 mm, the polymer was crushed with a meat chopper (MEAT-CHOPPER TYPE: 12VR-400KSOX, having die hole diameter: 9.5 mm, hole number: 18 and die thickness: 8 mm, manufactured by Iizuka Industry Co., Ltd.) at a charging rate of about 6 g/second, while ion-exchanged water was added thereto at a flow rate of 1.4 g/second, to yield a crushed hydrate gel-like cross-linked polymer.

(Drying, pulverizing and classification)

The finely crushed gel particles were spread onto a wire gauze with a mesh opening of 850 μm, dried with hot air at 180 °C for 40 minutes, pulverized by using a roll mill (WML-type roll pulverizing machine, manufactured by Inokuchi Giken, Ltd.), and classified by using JIS 850 μm and 150 μm standard sieves, to yield a water-absorbing resin (1) with irregularly crushed-shape, which has a D50 of 310 μm; a ratio of particles, with a particle diameter of equal to or larger than 300 μm and smaller than 600 μm, of 69% by weight; a ratio of particles, with a particle diameter of equal to or larger than 850 μm, of 0% by weight; a ratio of particles, with a particle diameter of smaller than 150 μm, of 0.1% by weight; a logarithmic standard deviation (σζ) of 0.334; and a solid content of 96% by weight. This water-absorbing resin (1) was used as a water-absorbing agent (1).

[Example 2]

By a similar method as in the Example 1, except that the amount of polyethylene glycol diacrylate (average adduct mole number of ethylene oxide: 9, average molecular weight: 522), as an internal cross-linking agent, was changed to 0.212 g (0.101% by mole (relative to the monomer)), a water-absorbing resin (2) with irregularly crushed-shape was obtained, which has a D50 of 315 $\mu$m; a ratio of particles, with a particle diameter of equal to or larger than 300 $\mu$m and smaller than 600 $\mu$m, of 71% by weight; a ratio of particles, with a particle diameter of equal to or larger than 850 $\mu$m, of 0% by weight; a ratio of particles, with a particle diameter of smaller than 150 $\mu$m, of 0.1% by weight; a logarithmic standard deviation ($\sigma\zeta$) of 0.321; and a solid content of 96% by weight. This water-absorbing resin (2) was used as a water-absorbing agent (2).

[Example 3]

By a similar method as in the Example 1, except that the 1.0% by weight acrylic acid solution of IRGACURE (registered trade name) 184 was not used, a water-absorbing resin (3) with irregularly crushed-shape was obtained, which has a D50 of 320 $\mu$m; a ratio of particles, with a particle diameter of equal to or larger than 300 $\mu$m and smaller than 600 $\mu$m, of 65% by weight; a ratio of particles, with a particle diameter of equal to or larger than 850 $\mu$m, of 0% by weight; a ratio of particles, with a particle diameter of smaller than 150 $\mu$m, of 0.1% by weight; a logarithmic standard deviation ($\sigma\zeta$) of 0.334; and a solid content of 97% by weight. This water-absorbing resin (3) was used as a water-absorbing agent (3).

[Comparative Example 1]

Into 5,500 g of an aqueous 38% by weight solution of acrylic acid having a neutralization ratio of 75% by mole, 2.094 g (0.017% by mole, relative to the monomer) of polyethylene glycol diacrylate (average adduct mole number of ethylene oxide: 9, average molecular weight: 522), as an internal cross-linking agent, was dissolved, to obtain a reaction solution. Then, the reaction solution was subjected to deaeration for 30 minutes under nitrogen gas atmosphere. Then, the reaction solution was supplied into a reactor having fitted with a lid, a jacketed stainless steel double arm-type kneader, with an inner volume of 10 liters and equipped with two sigma-type blades. The system was replaced with nitrogen gas, while maintaining the reaction solution at 25°C.

Subsequently, when 2.8 g of sodium persulfate as a polymerization initiator and 0.014 g of L-ascorbic acid were added to the reaction solution with stirred, polymerization was initiated after about 1 minute from the addition. Then, polymerization was performed at 25 to 95°C. The reaction was terminated after 40 minutes from initiation of the polymerization, to yield a hydrate, gel-like cross-linked polymer (1), which was crushed to a size of 1 to 5 mm. The hydrate, gel-like cross-linked polymer (1) thus finely crushed were spread onto a wire gauze with a mesh opening of JIS 300 $\mu$m, and dried with hot air at 180°C for 45 minutes. Subsequently, the dried substance was pulverized by using a roll mill (WML-type roll pulverizing machine, manufactured by Inokuchi Giken, Ltd.), and classified by using JIS 850 $\mu$m and 150 $\mu$m standard sieves, to yield a water-absorbing resin (C1) with irregularly crushed-shape, which has a D50 of 330 $\mu$m; a ratio of particles, with a particle diameter of equal to or larger than 300 $\mu$m and smaller than 600 $\mu$m, of 65% by weight; a ratio of particles, with a particle diameter of equal to or larger than 850 $\mu$m, of 0% by weight; a ratio of particles, with a particle diameter of smaller than 150 $\mu$m, of 0.2% by weight; a logarithmic standard deviation ($\sigma\zeta$) of 0.362; and a solid content of 95% by weight. This water-absorbing resin (C1) was used as a water-absorbing agent (C1).

[Comparative Example 2]

By a similar method as in the Comparative Example 1, except that the amount of polyethylene glycol diacrylate (average molecular weight: 522), as an internal cross-linking agent, was changed to 1.602 g (0.013% by mole (relative to the monomer)), a water-absorbing resin (C2) with irregularly crushed-shape was obtained, which has a D50 of 325 $\mu$m; a ratio of particles, with a particle diameter of equal to or larger than 300 $\mu$m and smaller than 600 $\mu$m, of 68% by weight; a ratio of particles, with a particle diameter of equal to or larger than 850 $\mu$m, of 0% by weight; a ratio of particles, with a particle diameter of smaller than 150 $\mu$m, of 0.1% by weight; a logarithmic standard deviation ($\sigma\zeta$) of 0.364; and a solid content of 95% by weight. This water-absorbing resin (C2) was used as a water-absorbing agent (C2).

[Comparative Example 3]

By a similar method as in the Comparative Example 1, except that the amount of polyethylene glycol diacrylate (average molecular weight: 522), as an internal cross-linking agent, was changed to 3.081 g (0.025% by mole (relative to the monomer)), a water-absorbing resin (C3) with irregularly crushed-shape was obtained, which has a D50 of 340

μm; a ratio of particles, with a particle diameter of equal to or larger than 300 μm and smaller than 600 μm, of 67% by weight; a ratio of particles, with a particle diameter of equal to or larger than 850 μm, of 0% by weight; a ratio of particles, with a particle diameter of smaller than 150 μm, of 0.1% by weight; a logarithmic standard deviation ($\sigma\zeta$) of 0.353; and a solid content of 95% by weight. This water-absorbing resin (C3) was used as a water-absorbing agent (C3).

[Comparative Example 4]

By a similar method as in the Comparative Example 1, except that the amount of polyethylene glycol diacrylate (average molecular weight: 522), as an internal cross-linking agent, was changed to 1.232 g (0.010% by mole (relative to the monomer)), a water-absorbing resin (C4) with irregularly crushed-shape was obtained, which has a D50 of 325 μm; a ratio of particles, with a particle diameter of equal to or larger than 300 μm and smaller than 600 μm, of 70% by weight; a ratio of particles, with a particle diameter of equal to or larger than 850 μm, of 0% by weight; a ratio of particles, with a particle diameter of smaller than 150 μ, of 0.1% by weight; a logarithmic standard deviation ($\sigma\zeta$) of 0.383; and a solid content of 95% by weight. This water-absorbing resin (C4) was used as a water-absorbing agent (C4).

Measurement results of absorbency, extractable content, weight average molecular weight and molecular weight distribution of main chains of the water-absorbing agent, obtained in each of the above Examples and Comparative Examples, are shown in the following Table 1.

Table 1

| | Water-absorbing agent | GV (A) [g/g] | GV (B) [g/g] | Increase rate [%] | Extractable content [wt%] | Mw of main chain | Mn of main chain | Mw/Mn [-] |
|---|---|---|---|---|---|---|---|---|
| Example 1 | Water-absorbing agent (1) | 65.9 | 84.1 | 27.6 | 31.9 | $1.42\times10^6$ | $6.65\times10^5$ | 2.1 |
| Example 2 | Water-absorbing agent (2) | 71.9 | 91.5 | 27.2 | 44.2 | $1.47\times10^6$ | $7.60\times10^5$ | 1.9 |
| Example 3 | Water-absorbing agent (3) | 67.7 | 88.2 | 30.3 | 32.5 | $1.43\times10^6$ | $6.65\times10^5$ | 2.2 |
| Comparative Example 1 | Water-absorbing agent (C1) | 63.7 | 74.1 | 16.3 | 39.5 | $1.32\times10^6$ | $3.30\times10^5$ | 4.0 |
| Comparative Example 2 | Water-absorbing agent (C2) | 67.0 | 78.5 | 17.2 | 46.9 | $1.37\times10^6$ | $3.45\times10^5$ | 4.0 |
| Comparative Example 3 | Water-absorbing agent (C3) | 53.7 | 68.3 | 27.2 | 29.3 | $1.30\times10^6$ | $3.53\times10^5$ | 3.7 |
| Comparative Example 4 | Water-absorbing agent (C4) | 63.9 | 74.7 | 16.9 | 58.0 | $1.38\times10^6$ | $3.59\times10^5$ | 3.8 |
| GV(A): Absorption capacity without load in artificial urine (A) | | | | | | | | |
| GV(B): Absorption capacity without load in artificial urine (B) | | | | | | | | |
| Increase rate: Increase rate of absorption capacity without load in artificial urine (B) relative to artificial urine (A) | | | | | | | | |
| Mw: Weight average molecular weight | | | | | | | | |
| Mn: Number average molecular weight | | | | | | | | |

It is noted from the results shown in Table 1 that the water-absorbing agents obtained in Examples 1 to 3 have narrower molecular weight distribution of the main chain, and larger increase rate of absorption capacity without load in artificial urine (B) relative to artificial urine (A), as compared with the water-absorbing agents obtained in Comparative Examples 1 to 4. From this result, it is shown that, according to the present invention, the water-absorbing agent which is capable of exerting excellent absorption characteristics even when composition of liquid to be absorbed, such as urine, varies, can be provided. In addition, it can be noted that sufficient evaluation results are not necessarily obtained by a conventional evaluation which has used only physiological saline (0.9% by weight artificial urine (A)).

[Evaluation of absorbing body]

(Method for preparation of absorbing body)

In order to evaluate performance as an absorbing body, an absorbing body for evaluation was prepared.
One part by weight of a water-absorbing agent 1, and 9 parts by weight of crushed wood pulp were dry-blended by using a mixer. Then, the resultant mixture was spread on a wire screen with 400 mesh (a mesh size of 38 $\mu$m), to form a web with a size having a diameter of 90 mm$\varphi$. Further, the web was pressed under a pressure of 196.14 kPa (2 kgf/cm$^2$) for one minute, to obtain an absorbing body for evaluation, with a basis weight of about 0.05 g/cm$^2$.

(Method for measurement of re-wet)

At the bottom of a SUS Petri dish with an inner diameter of 95 mm$\varphi$, the absorbing body for evaluation was spread. 20 ml of artificial urine (artificial urine (A) or artificial urine (B)) was poured on the absorbing body, to have the absorbing body absorbed for 30 minutes without any load. Then, 15 pieces of filter papers (product name: JIS P3801, No. 2, having a thickness of 0.26 mm, a diameter of 90 mm$\varphi$, and a retention particle diameter of 5 $\mu$m, manufactured by ADVANTEC Toyo Kaisya Ltd.) (weight: W4 (g)) and a weight with a diameter of 90 mm$\varphi$(weight: 1 kg) were put in this order and left to stand for one minute. Subsequently, the weight was removed, to measure a weight (W5 (g)) of the 15 pieces of filters to calculate re-wet according to the following formula 8.
$$[\text{Formula 8}]$$
$$\text{Re-wet (g)} = \text{w5 (g)} - \text{W4 (g)}$$

In the present evaluation, re-wet to artificial urine (A) (re-wet (A)), and re-wet to artificial urine (B) (re-wet (B)) were measured for the water-absorbing agent (1) obtained in Example 1, and the water-absorbing agent (C1) obtained in Comparative Example 1. Results are shown in the following Table 2.
Table 2

|  | Re-wet (A) (g) | Re-wet (B) (g) |
|---|---|---|
| Water-absorbing agent (1) | 3.8 | 2.8 |
| Water-absorbing agent (C1) | 3.8 | 3.3 |

It was noted from the results shown in Table 2 that the water-absorbing agent (1) of the Example of the present invention has lower re-wet (B) to artificial urine (B), which contains a polyvalent metallic ion (a magnesium ion or a calcium ion) and has a composition nearer to human urine, as compared with the water-absorbing agent (C1) of the Comparative Example, although re-wet (A) to the artificial urine (A), which is physiological saline (an aqueous 0.9% by weight sodium chloride solution) to be used in many conventional evaluations, was equivalent to the water-absorbing agent (C1).
It can be noted from these results that the water-absorbing agent of the present invention can exert excellent effects on reduction of slimy feeling or wet feeling in a state close to more practical use, such as in use of a diaper for children.
**Claims**

**1.** A particulate water-absorbing agent comprising a polyacrylate salt-type water-absorbing resin as a main component, and satisfying the following (a) and (b):

(a) absorption capacity without load in artificial urine (A) is equal to or higher than 62 g/g; and
(b) an increase rate of absorption capacity without load in artificial urine (B) relative to artificial urine (A) is equal to or larger than 20%,

wherein the polyacrylate salt-type water-absorbing resin is a cross-linked polymer containing acrylic acid (salt) in the repeating units thereof (excluding a cross-linking agent) in an amount of 70 to 100% by mole, and the acrylic acid (salt) as the repeating unit is neutralized in the range of 20 to 100% by mole in the form of an alkali metal salt, and wherein

artificial urine (A) is an aqueous 0.90% by weight sodium chloride solution, and artificial urine (B), a solution of 0.25 g of calcium chloride dihydrate, 2.0 g of potassium chloride, 0.50 g of magnesium chloride hexahydrate, 2.0 g of sodium sulfate, 0.85 g of ammonium dihydrogen phosphate, 0.15 g of diammonium hydrogen phosphate, and 994.25 g of pure water, and

wherein the absorption capacity without load in artificial urine (A) is measured as follows:

A water-absorbing agent passing through a 600 $\mu$m sieve but not passing through a 300 $\mu$m sieve was selected to use as a sample for measurement. Into a bag made of nonwoven fabric ("Heatron Paper GS-22" (85 mm x 60 mm), manufactured by Nangoku Pulp Industry Co., Ltd.), 0.20 g of the water-absorbing agent was uniformly charged, and after sealing, the bag was immersed in an artificial urine (A) for 30 minutes. Then, the bag was pulled up and drained off the water by using a centrifugal separator (a centrifugal separator: model H-122, manufactured by Kokusan Co., Ltd.) under a centrifugal force (250 G) described in EDANA Absorbency III441.1-99, for 3 minutes to weigh the bag, W1 (g). A similar process was performed without using the water-absorbing agent to weigh the bag, W0 (g).

Absorption capacity without load (g/g) in the artificial urine (A) was calculated according to the following formula 3

## Formula 3

Absorption capacity without load in artificial urine (A), (GV(A)) (g/g) =

$\{(W1 \ (g) - W0 \ (g)) / (\text{weight of water-absorbing agent (g)})\} - 1$

The absorption capacity without load in artificial urine (B) is measured by the method described above, using artificial urine (B) instead of artificial urine (A).

The bag was pulled up and drained off the water by using a centrifugal separator (a centrifugal separator: model H-122, manufactured by Kokusan Co., Ltd.) under a centrifugal force (250 G) described in EDANA Absorbency III441.1-99, for 3 minutes to weigh the bag, W3 (g). A similar process was performed without using the water-absorbing agent to weigh the bag, W2 (g). Absorption capacity without load (g/g) in the artificial urine (B) was calculated according to the following formula 4.

## Formula 4

Absorption capacity without load to artificial urine (B), (GV(B)) (g/g) =

$\{(W3 \ (g) - W2 \ (g)) / (\text{weight of water-absorbing agent (g)})\} - 1$

The increase rate of absorption capacity without load in artificial urine (B) relative to artificial urine (A) was calculated according to the following formula 5:

Formula 5

Increase rate of absorption capacity without load in artificial urine (B)
relative to artificial urine (A) [%] = 100 × {(absorption capacity without
load in artificial urine (B) (g/g)) - (absorption capacity without load in
artificial urine (A) (g/g))} / (absorption capacity without load in artificial
urine (A) (g/g))

**2.** A particulate water-absorbing agent according to claim 1 further satisfying the following (d):

(d) weight average molecular weight (Mw) of the main chain of the water-absorbing resin is equal to or higher than 1,000,000 and molecular weight distribution (Mw/Mn) thereof is equal to or smaller than 3.3,

wherein weight average molecular weight (Mw) of the main chain of the water-absorbing resin and molecular weight distribution (Mw/Mn) thereof are measured by the method and means described in the description in paragraph [0123] to [0124].

**3.** The particulate water-absorbing agent according to claim 1 or 2, wherein, in the particulate water-absorbing agent, ratio of particles having a particle diameter of equal to or larger than 300 $\mu$m and smaller than 600 $\mu$m is equal to or larger than 50% by weight,
wherein the particle size is measured with a standard sieve classification, JIS Z8801-1(2000) as described in the description in paragraph [0102].

**4.** The particulate water-absorbing agent according to any one of claims 1 to 3, which has an extractable content of 20 to 70% by weight,
wherein the extractable content is measured by the method and means described in the description in paragraph [0118] to [0121].

**5.** The particulate water-absorbing agent according to any one of claims 1 to 4,
wherein logarithmic standard deviation ($\sigma\zeta$) of particle size distribution is 0.20 to 0.45,
wherein the particle size is measured with a standard sieve classification, JIS Z8801-1(2000) as described in the description in paragraph [0102].

**6.** The particulate water-absorbing agent according to any one of claims 1 to 5, which satisfies the following (e) to (g):

(e) weight average particle diameter (D50) is 200 to 500 $\mu$m;
(f) an amount of particles having a particle diameter smaller than 150 $\mu$m as determined by a JIS standard sieve is 0 to 5% by weight; and
(g) an amount of particles having a particle diameter of equal to or larger than 850 $\mu$m as determined by a JIS standard sieve is 0 to 5% by weight,

wherein the particle size is measured with a standard sieve classification, JIS Z8801-1(2000) as described in description at paragraph [0102].

**7.** The particulate water-absorbing agent according to any one of claims 1 to 6, further comprising a chelating agent in an amount of 0.00001 to 10 parts by weight,
relative to 100 parts by weight of the water-absorbing resin.

**8.** The particulate water-absorbing agent according to any one of claims 1 to 7,
wherein the polyacrylate salt-type water-absorbing resin is a water-absorbing resin obtained by polymerization of a monomer under conditions satisfying the following (h) and (i):

(h) the monomer contains acrylic acid (a salt thereof) in an amount of 70 to 100% by mole as a monomer, and a cross-linking agent in an amount of 0.000 to 1.000% by mole relative to the monomer; and

(i) a concentration of an aqueous solution of the monomer is equal to or higher than 40% by weight,

wherein the polyacrylate salt-type water-absorbing resin is a cross-linked polymer containing acrylic acid (salt) in the repeating units thereof (excluding a cross-linking agent) in an amount of 70 to 100% by mole, and the acrylic acid (salt) as the repeating unit is neutralized in the range of 20 to 100% by mole in the form of an alkali metal salt.

**9.** The particulate water-absorbing agent according to any one of claims 1 to 8, wherein the polyacrylate salt-type water-absorbing resin is a water-absorbing resin obtained by cast polymerization of the aqueous solution of the monomer while controlling a polymerization initiation temperature at equal to or higher than 40°C, or a peak temperature in polymerization at equal to or higher than 100°C, and wherein the polyacrylate salt-type water-absorbing resin is a cross-linked polymer containing acrylic acid (salt) in the repeating units thereof (excluding a cross-linking agent) in an amount of 70 to 100% by mole, and the acrylic acid (salt) as the repeating unit is neutralized in the range of 20 to 100% by mole in the form of an alkali metal salt.

**10.** The particulate water-absorbing agent according to any one of claims 1 to 9, which comprises agglomerating with water or an aqueous solution, wherein the amount of water or an aqueous solution to be used is in the range of 0.5 to 20 parts by weight, relative to 100 parts by weight of solid content of the water-absorbing resin, and amount of the hydrophilic organic solvent is in the range of 0 to 10 parts by weight, relative to the water-absorbing resin mixture.

**Patentansprüche**

**1.** Ein partikelförmiges wasserabsorbierendes Mittel, das als einen Hauptbestandteil ein wasserabsorbierendes Harz vom Polyacrylatsalztyp aufweist und den folgenden (a) und (b) genügt:

(a) Absorptionskapazität ohne Last in künstlichem Urin (A) is gleich oder höher als 62 g/g; und
(b) eine Steigerungsrate der Absorptionskapazität ohne Last in künstlichem Urin (B) relativ zum künstlichen Urin (A) ist gleich oder größer als 20%,

wobei das wasserabsorbierende Harz vom Polyacrylatsalztyp ein vernetztes Polymer ist, das in seinen Wiederholungseinheiten (ausschließend ein Vernetzungsmittel) Acrylsäure (Salz) in einer Menge von 70 bis 100 Mol-% enthält, und das Acrylsäure (Salz) als Wiederholungseinheit im Bereich von 20 bis 100 Mol-% in der Form eines Alkalimetallsalzes neutralisiert ist, und wobei künstlicher Urin (A) eine wässrige 0,90 gewichtsprozentige Natriumchloridlösung ist und,
künstlicher Urin (B) eine Lösung von 0,25 g Calciumchloriddihydrat, 2,0 g Kaliumchlorid, 0,50 g Magnesiumchloridhexahydrat, 2,0 g Natriumsulfat, 0,85 g Ammoniumdihydrogenphosphat, 0,15 g Diammoniumhydrogenphosphat und 994,25 g reinem Wasser, und
wobei die Absorptionskapazität ohne Last in künstlichem Urin (A) folgendermaßen gemessen wird:

Ein wasserabsorbierendes Mittel, das ein 600 $\mu$m Sieb passiert, aber nicht durch ein 300 $\mu$m Sieb passt, wurde ausgewählt zur Verwendung als Probe für die Messung. 0,20 g des wasserabsorbierenden Mittels wurden gleichförmig in einen Beutel aus Faservliesstoff ("Heatron Paper GS-22" (85 mm x 60 mm), hergestellt von Nangoku Pulp Industry., Ltd.) gefüllt, und nach dem Versiegeln wurde der Beutel 30 Minuten lang in einen künstlichen Urin (A) getaucht. Danach wurde der Beutel herausgezogen und das Wasser unter Verwendung eines Zentrifugalseparators (ein Zentrufugalseparator: Modell H-122, hergestellt von Kokusan Co., Ltd) unter einer Zentrifugalkraft (250 G) abgeschieden, beschrieben in EDANAAbsorbency III441.1-99, 3 Minuten lang bis zur Wägung des Beutels, W1 (g). Ein ähnliches Verfahren wurde ohne Verwendung des wasserabsorbierenden Mittels durchgeführt zur Wiegung des Beutels, W0 (g).
Die Absorptionskapazität ohne Last (g/g) im künstlichen Urin (A) wurde gemäß der folgenden Formel 3 berechnet

Formel 3

Absorptionskapazität ohne Last in künstlichem Urin (A), $(GV(A))(g/g) =$

$\{(W1\ (g) - W0\ (g)) / (\text{Gewicht des wasserabsorbierenden Mittels (g)})\} - 1$

Die Absorptionskapazität ohne Last in künstlichem Urin (B) wurde nach der oben beschriebenen Methode gemessen unter Verwendung von künstlichem Urin (B) anstelle von künstlichem Urin (A).

Der Beutel wurde herausgezogen und das Wasser unter Verwendung eines Zentrifugalseparators (ein Zentrifugalseparator: Modell H-122, hergestellt von Kokusan Co., Ltd.), unter einer Zentrifugalkraft (250 G) abgeschieden, wie in EDANAAbsorbency III441.1-99 beschrieben, 3 Minuten lang bis zur Wägung des Beutels, W3 (g). Ein ähnliches Verfahren wurde durchgeführt ohne Verwendung des wasserabsorbierenden Mittels zur Wägung des Beutels, W2 (g). Die Absorptionskapazität ohne Last (g/g) im künstlichen Urin (B) wurde gemäß der folgenden Formel 4 berechnet.

## Formel 4

Absorptionskapazität ohne Last für künstlichen Urin (B), (GV(B))(g/g) =

{(W3 (g) − W2 (g)) / (Gewicht des wasserabsorbierenden Mittels (g))} − 1

Die Steigerungsrate der Absorptionskapazität ohne Last in künstlichem Urin (B) relativ zu künstlichem Urin (A) wurde gemäß der folgenden Formel 5 berechnet:

## Formel 5

Steigerungsrate der Absorptionskapazität ohne Last in künstlichem Urin (B), relativ zu künstlichem Urin (A)[%] = 100 x {(Absorptionskapazität ohne Last in künstlichem Urin (B) (g/g)) − (Absorptionskapazität ohne Last in künstlichem Urin (A) (g/g))} / (Absorptionskapazität ohne Last in künstlichem Urin (A) (g/g))

**2.** Partikelförmiges wasserabsorbierendes Mittel gemäß Anspruch 1, weiterhin genügend dem folgenden (d):

(d) Gewichtsmittel des Molekulargewichts (Mw) der Hauptkette des wasserabsorbierenden Harzes ist gleich oder größer als 1.000.000 und die Molekulargewichtsverteilung (Mw/Mn) ist gleich oder kleiner als 3,3,

wobei das Gewichtsmittel des Molekulargewichts (Mw) der Hauptkette des wasserabsorbierenden Harzes und die Molekulargewichtsverteilung (Mw/Mn) gemäß Verfahren und Mitteln, beschrieben in der Beschreibung in den Absätzen [0123] bis [0124] gemessen werden.

**3.** Das partikelförmige wasserabsorbierende Mittel nach Anspruch 1 oder 2, wobei im partikelförmigen wasserabsorbierenden Mittel der Anteil von Partikeln, die einen Partikeldurchmesser von gleich oder größer als 300 μm und kleiner als 600 μm haben, gleich oder größer als 50 Gewichtsprozent ist,
wobei die Partikelgröße mit einer Standardsiebklassifikation, JISZ8801-1(2000), wie in der Beschreibung im Paragraph [0102] beschrieben, gemessen wird.

**4.** Das partikelförmige wasserabsorbierende Mittel gemäß einem der Ansprüche 1 bis 3, welches einen Gehalt an extrahierbaren Verbindungen von 20 bis 70 Gewichtsprozent hat, wobei der Gehalt an extrahierbaren Verbindungen mittels eines Verfahrens und Mitteln gemessen wird, die in der Beschreibung in den Paragraphen [0118] bis [0121] beschrieben sind.

**5.** Das partikelförmige wasserabsorbierende Mittel gemäß einem der Ansprüche 1 bis 4, wobei die logarithmische Standardabweichung (σζ) der Partikelgrößenverteilung 0,20 bis 0,45 beträgt,
wobei die Partikelgröße mit einer Standardsiebklassifizierung JISZ8801-1(2000) gemessen wird, wie in der Beschreibung in Paragraph [0102] beschrieben.

**6.** Das partikelförmige wasserabsorbierende Mittel gemäß einem der Ansprüche 1 bis 5, welches den folgenden (e) bis (g) genügt:

(e) Gewichtsmittel des Partikeldurchmessers (D50) ist 200 bis 500 $\mu$m;

(f) ein Gehalt an Partikeln, welche einen Partikeldurchmesser kleiner als 150 $\mu$m haben, bestimmt nach JIS Standardsieb, ist 0 bis 5 Gewichtsprozent; und

(g) ein Gehalt an Partikeln, welche einen Partikeldurchmesser von gleich oder größer als 850 $\mu$m haben, bestimmt nach JIS Standardsieb, ist 0 bis 5 Gewichtsprozent, wobei die Partikelgröße mit einer Standardsiebklassifizierung JISZ8801-1(2000) gemessen wird, wie in der Beschreibung im Absatz 102 beschrieben.

7. Das partikelförmige wasserabsorbierende Mittel gemäß einem der Ansprüche 1 bis 6, weiterhin aufweisend ein Komplexierungsmittel in einer Menge von 0,00001 bis 10 Gewichtsteilen, bezogen auf 100 Gewichtsteile des wasserabsorbierenden Harzes.

8. Das partikelförmige wasserabsorbierende Mittel gemäß einem der Ansprüche 1 bis 7, wobei das wasserabsorbierende Harz vom Polyacrylatsalztyp ein wasserabsorbierendes Harz ist, erhalten durch Polymerisation eines Monomers unter Bedingungen, die den folgenden (h) und (i) genügen:

(h) das Monomer enthält Acrylsäure (ein Salz davon) in einer Menge von 70 bis 100 Mol-% als ein Monomer und ein Vernetzungsmittel in einer Menge von 0,000 bis 1,000 Mol-%, bezogen auf das Monomer; und
(i) eine Konzentration der wässrigen Lösung des Monomers ist gleich oder höher als 40 Gewichts-%,

wobei das wasserabsorbierende Harz vom Polyacrylatsalztyp ein vernetztes Polymer ist, welches das Acrylsäure (Salz) in seinen Wiederholungseinheiten (ausschließend ein Vernetzungsmittel) in einer Menge von 70 bis 100 Mol-% enthält, und das Acrylsäure (Salz) als Wiederholungseinheit im Bereich von 20 bis 100 Mol-% in Form eines Alkalimetallsalzes neutralisiert ist.

9. Das partikelförmige wasserabsorbierende Mittel gemäß einem der Ansprüche 1 bis 8, wobei das wasserabsorbierende Harz vom Polyacrylatsalztyp ein wasserabsorbierendes Harz ist, welches durch Gießpolymerisation einer wässrigen Lösung des Monomers erhalten wurde, während man die Polymerisationsinitiierungstemperatur bei gleich oder größer als 40°C oder eine Spitzentemperatur bei der Polymerisation auf gleich oder größer als 100°C kontrolliert, und wobei das wasserabsorbierende Harz vom Polyacrylatsalztyp ein vernetztes Polymer ist, das Acrylsäure (Salz) in den Wiederholungseinheiten (ausschließend ein Vernetzungsmittel) in einer Menge von 70 bis 100 Mol-% enthält, und das Acrylsäure (Salz) als Wiederholungseinheit im Bereich von 20 bis 100 Mol-% in Form eines Alkalimetallsalzes neutralisiert ist.

10. Das partikelförmige wasserabsorbierende Mittel gemäß einem der Ansprüche 1 bis 9, welches eine Agglomeration mit Wasser oder einer wässrigen Lösung umfasst, wobei die Menge an Wasser oder einer wässrigen Lösung, die eingesetzt wird, im Bereich von 0,5 bis 20 Gewichtsteilen liegt, bezogen auf 100 Gewichtsteile des Feststoffgehaltes des wasserabsorbierenden Harzes, und der Gehalt des hydrophilen organischen Lösungsmittels im Bereich von 0 bis 10 Gewichtsteilen liegt, bezogen auf die wasserabsorbierende Harzmischung.

## Revendications

1. Absorbant d'eau granulaire comprenant une résine polyacrylate d'absorption d'eau de type salin constituant un composant principal, et répondant aux conditions (a) et (b) ci-dessous :

(a) la capacité d'absorption sans charge dans une urine artificielle (A) est égale ou supérieure à 62 g/g ; et
(b) un taux d'accroissement de la capacité d'absorption sans charge dans une urine artificielle (B) par rapport à l'urine artificielle (A) est égale ou supérieure à 20%,

dans lequel la résine polyacrylate d'absorption d'eau de type salin est un polymère réticulé contenant de l'acide acrylique (sel) dans les motifs répétés de celui-ci (à l'exception d'un agent de réticulation) en une quantité allant de 70 à 100% molaire, et l'acide acrylique (sel) constituant le motif répété est neutralisé dans la plage allant de 20 à 100% molaire sous la forme d'un sel de métal alcalin, et dans lequel
l'urine artificielle (A) est une solution aqueuse de chlorure de sodium 0,90% en poids, et
l'urine artificielle (B) est une solution de 0,25 g de dihydrate de chlorure de calcium, 2,0 g de chlorure de potassium, 0,50 g d'hexahydrate de chlorure de magnésium, 2,0 g de sulfate de sodium, 0,85 g de dihydrogénophosphate d'ammonium, 0,15 g de dihydrogénophosphate de diammonium, et 994,25 g d'eau pure, et
dans lequel la capacité d'absorption sans charge dans l'urine artificielle (A) est mesurée de la manière suivante :

un absorbant d'eau traversant un tamis 600 μm mais ne traversant pas un tamis 300 μm est sélectionné pour servir d'échantillon de mesure. Dans un sac constitué de non-tissé (« Heatron Paper GS-22 » (85 mm x 60 mm), produit par Nangoku Pulp Industry Co., Ltd.), 0,20 g de l'absorbant d'eau est uniformément chargé, et après avoir été scellé, le sac est immergé dans une urine artificielle (A) pendant 30 minutes. Ensuite, le sac est retiré et drainé de l'eau au moyen d'un séparateur centrifuge (un séparateur centrifuge : modèle H-122, produit par Kokusan Co., Ltd.) sous une force centrifuge (250 G) décrit dans EDANA Absorbency III441.1-99, pendant 3 minutes pour peser le sac, W1(g). Un procédé identique est réalisé sans utiliser l'absorbant d'eau pour peser le sac, W0(g).

La capacité d'absorption sans charge (g/g) dans l'urine artificielle (A) est calculée selon la formule suivante 3.

Formule 3

Capacité d'absorption sans charge dans une urine artificielle (A), (GV(A)) (g/g) = {(W1(g)-W0(g))/(poids de l'absorbant d'eau(g))}-1

La capacité d'absorption sans charge dans l'urine artificielle (B) est mesurée selon le procédé décrit ci-dessus, en utilisant l'urine artificielle (B) au lieu de l'urine artificielle (A).

Le sac est retiré et drainé de l'eau au moyen d'un séparateur centrifuge (un séparateur centrifuge : modèle H-122, produit par Kokusan Co., Ltd.) sous une force centrifuge (250 G) décrit dans EDANA Absorbency III441.1-99, pendant 3 minutes pour peser le sac, W3(g). Un procédé identique est réalisé sans utiliser l'absorbant d'eau pour peser le sac, W2(g). La capacité d'absorption sans charge (g/g) dans l'urine artificielle (B) est calculée selon la formule 4 suivante.

Formule 4

Capacité d'absorption sans charge dans l'urine artificielle (B), (GV(B)) (g/g) = {(W3(g)-W2(g))/(poids de l'absorbant d'eau(g))}-1

Le taux d'accroissement de la capacité d'absorption sans charge dans l'urine artificielle (B) par rapport à l'urine artificielle (A) est calculé selon la formule 5 suivante :

Formule 5

Taux d'accroissement de la capacité d'absorption sans charge dans l'urine artificielle (B) par rapport à l'urine artificielle (A) [%} = 100 x {(capacité d'absorption sans charge dans l'urine artificielle (B)(g/g))-(capacité d'absorption sans charge dans l'urine artificielle (A)(g/g))}/(capacité d'absorption sans charge dans l'urine artificielle (A)(g/g))

2. Absorbant d'eau granulaire selon la revendication 1 répondant en outre à la condition (d) ci-dessous :

(d) le poids moléculaire moyen en poids (Mw) de la chaine principale de la résine d'absorption d'eau est égal ou supérieur à 1.000.000 et la distribution en poids moléculaire (Mw/Mn) correspondante est égale ou inférieure à 3,3,

dans lequel le poids moléculaire moyen en poids (Mw) de la chaine principale de la résine d'absorption d'eau et la distribution en poids moléculaire (Mw/Mn) correspondante sont mesurés selon le procédé et les moyens décrits dans la description au paragraphe [0123] à [0124].

3.  Absorbant d'eau granulaire selon la revendication 1 ou selon la revendication 2, dans lequel, dans l'absorbant d'eau granulaire, le rapport de particules ayant un diamètre de particules égal ou supérieur à 300 μm et inférieur à 600 μm est égal ou supérieur à 50% en poids,
dans lequel la taille de particules est mesurée au moyen d'un tamis de classification standard JIS Z8801-1(2000) tel que décrit dans la description au paragraphe [0102].

4.  Absorbant d'eau granulaire selon l'une quelconque des revendications 1 à 3, qui présente une teneur extractible de 20 à 70% en poids,
dans lequel la teneur extractible est mesurée selon le procédé et les moyens décrits dans la description au paragraphe [0118] à [0121].

5.  Absorbant d'eau granulaire selon l'une quelconque des revendications 1 à 4, dans lequel la déviation standard logarithmique (σζ) de la distribution de tailles de particules est de 0,20 à 0,45,
dans lequel la taille de particules est mesurée au moyen d'un tamis de classification standard JIS Z8801-1(2000) tel que décrit dans la description au paragraphe [0102].

6.  Absorbant d'eau granulaire selon l'une quelconque des revendications 1 à 5, qui répond aux conditions (e) à (g) ci-dessous :

(e) le diamètre de particules moyen en poids (D50) est de 200 à 500 μm ;
(f) une quantité de particules ayant un diamètre de particules inférieur à 150 μm tel que déterminé au moyen d'un tamis standard JIS est de 0 à 5% en poids ; et
(g) une quantité de particules ayant un diamètre de particules égal ou supérieur à 850 μm tel que déterminé au moyen d'un tamis standard JIS est de 0 à 5% en poids,

dans lequel la taille de particules est mesurée au moyen d'un tamis de classification standard JIS Z8801-1(2000) tel que décrit dans la description au paragraphe [0102].

7.  Absorbant d'eau granulaire selon l'une quelconque des revendications 1 à 6, comprenant en outre un agent chélatant en une quantité allant de 0,00001 à 10 parties en poids, par rapport à 100 parties en poids de la résine d'absorption d'eau.

8.  Absorbant d'eau granulaire selon l'une quelconque des revendications 1 à 7, dans lequel la résine polyacrylate d'absorption d'eau de type salin est une résine d'absorption d'eau obtenue par polymérisation d'un monomère sous les conditions répondant aux conditions (h) et (i) ci-dessous :

(h) le monomère contient de l'acide acrylique (un sel de celui-ci) en une quantité allant de 70 à 100% molaire sous la forme d'un monomère, et un agent de réticulation en une quantité allant de 0,000 à 1,000% molaire par rapport au monomère ; et
(i) une concentration en solution aqueuse du monomère égale ou supérieure à 40% en poids,

dans lequel la résine polyacrylate d'absorption d'eau de type salin est un polymère réticulé contenant de l'acide acrylique (sel) dans les motifs répétés (à l'exception d'un agent de réticulation) en une quantité allant de 70 à 100% molaire, et l'acide acrylique (sel) constituant le motif répété est neutralisé dans la plage allant de 20 à 100% molaire sous la forme d'un sel de métal alcalin.

9.  Absorbant d'eau granulaire selon l'une quelconque des revendications 1 à 8, dans lequel la résine polyacrylate d'absorption d'eau de type salin est une résine d'absorption d'eau obtenue par polymérisation par coulée de la solution aqueuse du monomère tout en contrôlant une température d'initiation de polymérisation à une température égale ou supérieure à 40°C, ou une température de pointe au cours de la polymérisation égale ou supérieure à 100°C, et dans lequel la résine polyacrylate d'absorption d'eau de type salin est un polymère réticulé contenant de l'acide acrylique (sel) dans les motifs répétés de celui-ci (à l'exception d'un agent de réticulation) en une quantité allant de 70 à 100% molaire, et l'acide acrylique (sel) constituant le motif répété est neutralisé dans la plage allant de 20 à 100% molaire sous la forme d'un sel de métal alcalin.

10. Absorbant d'eau granulaire selon l'une quelconque des revendications 1 à 9, qui comprend l'agglomération avec de l'eau ou une solution aqueuse, dans lequel la quantité d'eau ou de solution aqueuse utilisée est dans la plage allant de 0,5 à 20 parties en poids, par rapport à 100 parties en poids de la teneur solide de la résine d'absorption l'eau, et la quantité de solvant organique hydrophile est dans la plage allant de 0 à 10 parties en poids, par rapport au mélange de résine d'absorption l'eau.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20050221980 A
- US 6187872 B
- JP 2006122737 A
- US 6586549 B
- US 6906159 A
- US 6906159 B2
- US 2004110897 A
- US B670141 A
- US 4625001 B
- US 5250640 B
- US 4093776 B
- US 4367323 B
- US 4446261 B
- US 4683274 B
- US 5244735 B
- US 4873299 B
- US 4286082 B
- US 4973632 B
- US 4985518 B
- US 5124416 B
- US 5264495 B
- US 5145906 B
- US 5380808 B
- EP 0811636 B
- EP 0955086 B
- EP 0922717 B
- US 2005215734 A
- WO 200469915 A
- US 6297319 B
- US 6372852 B
- US 625488 B
- US 6809158 B
- US 4734478 B
- US 4755562 B
- US 4824901 B
- US 6239230 B
- US 6559239 B
- US 6472478 B
- US 6657015 B
- US 5672633 B
- US 6605673 B
- US 6620899 B
- US 2004106745 A
- US 5610208 B
- US 200548221 A
- US 2005118423 A
- US 6562879 B
- US 5744564 B
- WO 2005092956 A
- WO 200509956 A